# EUROPEAN PATENT SPECIFICATION

(11) **EP 1 957 083 B1**
(45) Date of publication and mention of the grant of the patent: **15.02.2012**
(21) Application number: 06809778.1
(22) Date of filing: 23.10.2006
(51) Int. Cl.: A61K 31/70, A61P 37/00

(54) **BETA GLYCOLIPIDS AS IMMUNO-MODULATORS**
BETA-GLYCOLIPIDE ALS IMMUNOMODULATOREN
BETA-GLYCOLIPIDES EN TANT QU'IMMUNOMODULATEURS

(30) Priority: 24.11.2005 IL 17217505
(43) Date of publication of application: 20.08.2008
(73) Proprietor: ENZO BIOCHEM, INC., New York, NY 10022 (US)
(72) Inventor: ILAN, Yaron, 96400 Jerusalem (IL)
(74) Representative: Barth, Renate
(86) International application number: PCT/IL2006/001217
(87) International publication number: WO 2007/060652

(56) References cited:
- EP-A- 0 719 787
- EP-A- 1 452 181
- WO-A-00/24406
- WO-A-2005/014008

## Description

### Field of the Invention

The invention relates to the use of β-glycolipids as immunomodulators. More particularly, the invention relates to the use of β-lactosyl-ceramide and β-glucosylceramide,

### Background of the Invention

Immune therapy involves the exposure of components of the immune system to various elements (cytokines, disease associated antigens and natural metabolites) to combat disease processes in which a dysregulated immune response is thought to play a role. Immune dysregulation is thought to play a major part in the pathogenesis or disease course of a great number of disease processes, including various neoplastic, inflammatory, infectious and genetic entities.

These disorders can be perceived as a dysbalance between pro-inflammatory (This) and anti-inflammatory (Th2) cytokines, and few of them are described herein below.

### The role of the immune system in the pathogenesis of inflammatory bowel disease

Inflammatory bowel diseases (IBD) are common gastrointestinal disorders, that can be perceived as being the result of a dysbalance between Th1-pro-inflammatory, and Th2-anti-inflammatory subtypes of immune responses [Strober, W., et al., Immunol Today 18:61-64 (1997); Neurath, M., et al., J. Exp. Med. 183:2605-2616 (1996)].

There are several extra-intestinal manifestations that accompany IBD, for example: autoimmune phenomena; immune complexes have a role in target organ damage; and, immunosuppressive agents such as glucocorticoids, azathioprine, methotrexate and cyclosporin are used to alleviate the disease [Podolsky, D.K., et al., New Engl. J. Med., 325:928-935(1991); Strober, W., et al., In Clinical Immunology, Mosby, St. Louis. R.R. Rich, Editor, 1401-14281-2 (1995)]. Patients with IBD have antibodies against components of colon cells and several different bacterial antigens. These antigens gain access to the immune system as a consequence of epithelial damage [Hibi, S., et al., Clin. Exp. Immunol. 54:163-168 (1983); Das, K.M., et al., Gastroenterology 98:464-69 (1990)]. Abnormalities of T cell-mediated immunity, including coetaneous anergy and diminished responsiveness to T cell stimuli, have also been described in these patients [Chiba, M., et al. Gut, 22:177-182 (1981); Raedler, A., et al., Clin. Exp. Immunol. 60:518-526 (1985)]. In addition, changes in mucosal cell mediated immunity were identified, including increased concentrations of mucosal IgG cells and changes in T cells subsets, suggesting, antigen stimulation [Dasgupta, A., et al., Gut 35:1712-17 (1994); Takahashi, F., et al., J. Clin. Invest. 76:311-318 (1985)]. Exposure of target antigens after infectious, immune, or toxic damage, leads to activation of mucosal immune cells resulting in cytokines that lead to mucosal inflammatory response [Neurath, M., et al., J. Exp. Med., 183:2605-2616 (1996)]. Secretion of pro-inflammatory cytokines such as IFNγ, contributes to an increase in mucosal permeability, and has been described in animal models of IBD [Strober, W., et al., Immunol. Today 18:61-64. (1997)]. Similarly, an increase in collagen synthesis mediated by IL1 and IL6 can be detected in these animals [Strober, W., *et al*., *ibid*.]. A Thi-mediated granulomatous colitis model has been established by the adoptive transfer of normal CD45RB T cells from Balb/C mice into CB-17 scid mice. CD4 cells from CD45RB were shown to prevent the disease when injected together with the CD45RB population. This prevention could be reversed by adding antibodies to TGFβ1 [Sadlack, B., et al., Cell 75:253-261 (1993); Powrie, F., et al., Immunity 1:553-562 (1994)].

### The Th1/Th2 dysbalance in inflammatory bowel disease

Both CD4 and CD8 lymphocytes can be typed as either Th1 cells that produce IL-2 and IFNγ, or Th2 cells that produce IL-4, and IL-10. The way the immune system responds to foreign and self antigens, is the result of a balance between the two subtypes of responses [Weiner, H.L., et al., Immunol. Today 18: 335-343 (1997); Adorini, L., et al., Immunol. Today 18:209-211 (1997)]. A Th1 type response is involved in the pathogenesis of several autoimmune and chronic inflammatory disorders such as IBD [Adorini, L., *et al.,* (1997) *ibid.;* Mizoguchi, A., et al., J. Exp. Med. 183:847-856, (1996)]. Thus experimental colitis and IBD in humans can be perceived as a dysbalance between pro-inflammatory Th1-type and anti-inflammatory Th2-type cytokines. It has been recently shown, in both animals and humans, that anti-inflammatory cytokines such as IL10 can downregulate the pro-inflammatory effects of Th1-mediated cytokines, thereby alleviating immune-mediated disorders [Mizoguchi, A., *et al.,* (1996) *ibid.;* Madsen, K.L., et al., Gastroenterology 113:151-159 (1997); Van Deventer Sander, J., et al., Gastroenterology 113:383-389 (1997)].

### The Role of the Immune System in the Pathogenesis of Non-Alcoholic Steatohepatitis

Non-alcoholic steatohepatitis (NASH) is a clinico-pathological entity consisting of hepatic fat accumulation, inflammation and fibrosis in patients who have no history of alcohol consumption. It may progress to cirrhosis in 20% of cases and is considered the most common cause of cryptogenic cirrhosis in the Western world [Caldwell, S.H. et al., Hepatology 29:664 (1999); Matteoni, C.A. et al., Gastroenterology 116:1413 (1999)]. NASH is common in patients who suffer of other metabolic disturbances, which are suggested to play a contributing role in the pathogenesis of the disorder. These include insulin resistance [Sanyal, A.J. et al., Gastroenterology 120:1183 (2001)], obesity-related ATP depletion [Cortez-Pinto, H. et al., Jama 282:1659 (1999)], increased free-fatty-acid beta peroxidation [Hruszkewycz, A.M. Biochem. Biophys. Res. Commun. 153:191 (1988)], iron accumulation [George, D.K. et al., gastroenterology 114:311 (1998)], antioxidant depletion [Harrison, S.A. et al., gastroenterology 123:M332 (2002)], and leptin deficiency [Cohen, B. et al., Science 274:1185 (1996)]. Yet no therapeutic intervention, including weight loss, tight diabetic control, normalization of lipid levels and antioxidant treatment have consistently shown an alteration in the natural progression of the disorder [Angulo, P. New England Journal of Medicine 346:1221-1231 (2002)].

Most information about NASH has been derived from two mammalian models: leptin-deficient ob/ob mice and leptin-receptor deficient fa/fa Zucker rats. Leptin is a protein that is involved with the regulation of body weight [Zhang, Y. et al., Nature 372:425-432 (1994)]. Its deficiency in rodents and humans results in a severe form of 'metabolic syndrome' (formerly termed syndrome X) consisting of morbid obesity, glucose intolerance, hyperlipidemia, and severe hepatic steatosis [Pelleymounter, M.A., et al., Science 269:540-543 (1995)]. Yet, as mentioned above, no intervention aimed at correcting some of these metabolic disturbances have resulted in an amelioration of the hepatic steatosis, fibrosis, and inflammation.

Recent evidence suggests that the immune system may play a pivotal role in the pathogenesis of NASH in the leptin deficient models. In leptin deficient mice, defective hepatic macrophage (Kupffer cell) response has been observed after liver injury induction by lipopolysaccharide [Diehl, A.M. J. Physiol. Gastrointest. liver Physiol. 282:G1-G5 (2002)]. In similar models, LPS induction of IL6 was greatly enhanced, while that of IL10 was inhibited [Loffreda, S, et al., FASEB J. 12:57-65 (1998)]. Ob/ob mice hepatic macrophages were observed to produce more IL12 and less IL15 than control mice in response to LPS challenge, which may explain the significant reduction in the number and function of NKT lymphocytes observed in these mice [Yang et al., Proc Natl Acad Sci USA 94:2557-2562 (1997)]. Other observations have shown a reduction in the number of CD4 T lymphocytes in the blood and liver of leptin-deficient ob/ob mice [Howard, J.K. et al, J. Clin. Invest. 104:1051-1059 (1999) and Lord, et al., Nature 394:897-901 (1998)]. This may explain the relative resistance of leptin-deficient mice to Concanavalin A hepatitis, which is mediated by CD4 T lymphocytes [Faggioni, R. et al., Proc. Natl. Acad. Sci. USA 97:2367-2372 (2000)].

### The Th1 /Th2 Dysbalance in Non-Alcoholic Steatohepatitis

CD4 and CD8 lymphocytes are classified as either Th1 cells that produce IL-2 and IFNγ, or Th2 cells that produce IL-4 and IL-10. The immune system responds to foreign and self-antigens by a shift in balance between the two subtypes of responses [Weiner, H.L. et al., Immunol. Today 18: 335-343 (1997); Adorini, L. et al., Immunol. Today 18:209-211 (1997)]. Usually the Th1 type response causes a pro-inflammatory reaction [Adorini, L. *et al.,* (1997) *ibid.;* Mizoguchi, A., et al., J. Exp. Med. 183:847-856, (1996)], while anti-inflammatory cytokines such as IL10 shift the balance towards an anti-inflammatory Th2 reaction, thereby alleviating immune-mediated disorders [Mizoguchi, A. *et al.,* (1996) *ibid.;* Madsen, K.L. et al., Gastroenterology 113:151-159 (1997); Van Deventer Sander, J. et al., Gastroenterology 113:383-389 (1997)]. NKT cells, in response to different endogenous and exogenous stimuli, are believed to play a major role in the direction of the immune system towards either the Th1 or Th2 pathways.

Leptin has been shown to play a major role in the immune regulation of the balance between Th1 & Th2 response (Lord, G.M. et al., Nature 394:897-901 (1998)]. In the leptin-deficient ob/ob mice NASH model an alteration of the number and function of NKT cells has been suggested to tilt the immune system towards the Th1 response. This is suggested to result in an increased sensitivity to LPS induced hepatotoxicity and a unique resistance to the hepatotoxic effects of Concanavalin A. The difference may be in their different pathogenic mechanisms. The former depends upon the action of the innate hepatic immune system, which is hyperactive in the leptin-deficient mice, while the latter is dependent upon the activation of NKT-lymphocytes, which are suppressed and defective in the leptin deficient mice [Faggioni, R. et al., PNAS 97:2367-2372 (2000), Zhiping, L.I. et al., Gastroenterology 123:1304-1310 (2002)].

### The Immune System and Obesity

The immune system and the regulation of adipose tissue metabolism appear to be closely interlinked. Up to fifty percent of cells within adipose tissues are composed of non-adipose cells, including many immunocytes [Montague, C.T. et al., Diabetes 47:1384-91 (1998)]. Most research has been focused on the immunological consequences of morbid obesity. Immunological alterations which are known to exist in obese animals and humans included reduced DTH and mitogen-stimulated lymphocyte proliferation responses [Chandra, R.K. et al., Acta. Paediatr. Scand 69:25-30 (1980)], impaired phagocyte number and function [Krishnan, E.C. et al., J. Surg. Res. 33:89-97 (1982)], attenuation of insulin induced lymphocyte cytotoxicity [Koffler, M. et al., Diabetes 40:364-360 (1991)], and changes in the CD4/CD8 ratio, especially during weight loss attempts [Field, C.J. et al., Am. J. Clin. Nutr. 54:123-129 (1991)].

Adipose cells are known to secrete pro-inflammatory cytokines including TNF-β [Hotamisligil, G.S. et al., Science 259:87-91 (1993)] and IL6 [Purohit, A. et al., Journal of Clinical Endocrinology and Metabolism 80:3052-58 (1995)], which are both related to the level of adiposity [Hotamisligil, G.S. et al., Journal of Internal Medicine 245:621-625 (1999)]. Some of these cytokines are considered to have metabolic effects such as insulin resistance mediated by TNF- β [Ogawa, H. et al., Biochimica et biophysica acta 1003:131-135 (1989)] and lipoprotein lipase inhibition mediated by IL6 [Feingold, et al., Diabetes 41:97s-101s (1992)]. TNF- β knockout mice have higher insulin sensitivity and improved lipid profile than their normal littermates [Uysal, et al., Nature 389:610-614 (1997)]. Other components of the immune system, which are produced by adipose cells, include the protein adipsin, which is an integral part of the alternative complement system, and functions identically to human complement factor D [Rosen, B.S. et al., Science 244:1483-7 (1989)].

Little information is known about the role of the immune system as a mediator of obesity, but several recent studies suggest that the immune system may have an important contributory role in the development of obesity. Several cytokines are known to act as adipose tissue regulators. TNF- β suppresses the expression of β₃ adreno-receptors on adipose cells, which are involved in sympathetically mediated lipolysis, while IL1 stimulates adipose leptin secretion [Sarraf, et al., Journal of experimental medicine 185:171-175 (1997)]. The metabolic activity rate of adipose cells has been observed to be closely correlated to their distance from the closest lymph node [Pond, C.M. et al., Proceedings of the nutrition society 60:365-374 (2001)], through a mechanism which is partly mediated by IL4, IL6 and TNF- β [Mattacks, C.A. et al., Cytokine 11:334-346 (1999)].

These observations, which point to the fact that obese animals and humans may also be suffering of various alterations in the different arms of the immune system, suggest that modulation of the immune system may change some of the pathogenic mechanisms responsible for the development of morbid obesity.

To the best of the inventor's knowledge, previously employed methods of immune modulation have not involved exposure of components of the immune system to mammalian naturally occurring β-glycolipids and specifically to a mixture of β-glycolipids.

WO 2005/032462, which is a previous publication by the present inventors, discloses the general use of intermediary metabolites and preferably, glucocerebrosides, in the treatment of immune-related disorders. The present invention now clearly shows that certain intermediary metabolites, the β-glycolipids and not the α-glycolipids are particularly effective, and specifically, β-lactosyl-ceramide (LacC), β-glucosylceramide (GluC), and β-galactosyl-ceramide (GalC)] and ceramide. Surprisingly, the inventors have now showed for the first time that β-lactosyl-ceramide may be used as a preferred β-glycolipid for immune-modulation.

Moreover, the inventors show a clear synergistic effect of a particular combination of two β-glycolipids, preferably a mixture of β-lactosyl-ceramide with β-glucosylceramide, which may be used as a powerful medicament for the treatment of immune-related disorders.

These and other objects of the invention will become clearer as the description proceeds

### Summary of the Invention

As a first aspect, the present invention relates to a process for the modulation of the Th1/Th2 cell balance toward anti-inflammatory cytokine producing cells, in a subject suffering from an immune related disorder. This process comprises the step of increasing the intracellular, extra-cellular or serum level of a naturally occurring β-glycolipid in a subject in need thereof. The modulation of the Th1/Th2 cell balance may be mediated by at least one component of said subject immune system. According to this embodiment, increasing the intracellular, extra-cellular or serum level of a naturally occurring β-glycolipid in said subject may be performed by:
(I) administering an effective amount of any one of: a β-glycolipid, a mixture of at least two β-glycolipids, a substance which increases the intracellular, extracellular or serum level of a naturally occurring β-glycolipids, and any combination of the above; or
(II) exposing at least one component of said subject immune system to an effective amount of any one of: a mixture of at least two β-glycolipids: as claimed

In a second aspect, the invention relates to a method for the treatment of immune-related disorder in a mammalian subject in need thereof. According to one embodiment, the method of treatment comprises the step of administering to said subject an effective amount of a mixture of at least two naturally occurring β-glycolipids

The invention further provides a method for the treatment of immune-related disorder in a mammalian subject in need thereof. According to this preferred embodiment, the method of the invention comprises the step of increasing the intracellular, extra-cellular or serum level of a naturally occurring β-glycolipid in said subject, by exposing at least one component of said subject immune-system to an effective amount of a mixture of at least two β-glycolipids

In a third aspect, the invention relates to a therapeutic composition for the treatment of an immune-related disorder in a mammalian subject. According to one embodiment, the composition of the invention may comprise as an active ingredient: (a) a mixture of at least two β-glycolipids, (b) antigens associated with said immune-related disorder (c) at least one of liver-associated cells of tolerized or non-tolerized subjects suffering from said immune-related disorder or of said subject (d) at least one of cytokines, adhesion molecules or any combination thereof (e) antigen presenting cells and (f) a combination of any of (a), (b), (c), (d) and (e).

According to another preferred embodiment, the therapeutic composition of the invention may comprise as an active ingredient, educated NK T cells capable of modulating the Th1/Th2 cell balance. More specifically, the educated NK T cells comprised within the composition of the invention were cultured in the presence of any one of: (a) a mixture of at least two β-glycolipids (b) antigens associated with said immune-related disorder (c) at least one of liver-associated cells of tolerized or non-tolerized subjects suffering from said immune-related disorder or of said subject (d) at least one of cytokines, adhesion molecules and any combination thereof (e) antigen presenting cells, and (f) a combination of any of (a), (b), (c), (d) and (e).

These and other aspects of the invention will become apparent by the hand of the following figures and examples.

### Brief Description of the Figures

**Figure 1****:** Effect of glycolipids on pathology score of TNBS colitis induced mice model. Abbreviations: sc. (score), Ext. (Extent), Inflam. (inflammation), Dam. (damage), Reg. (regeneration), OP (oral).
**Figure 2****:** Effect of glycolipids on serum cytokine levels of TNBS colitis induced mice model. Abbreviations: OP (oral), pg/ml (pictogram/milliliter).
**Figure 3****:** Effect of glycolipids on T lymphocyte distribution, of TNBS colitis induced mice model. Abbreviations: OP (oral), Per. (peripheral), Liv. (liver), Rat. (ratio).
**Figure 4****:** Effect of treatment with combination of GC and LC on macroscopic score of colitis. Abbreviations: Dia (diarrhea), Ulc. (ulcers), Ery. (erythema), Th. (thickness), Ad. (adhesion).
**Figure 5****:** Effect of treatment with different ratios of combination of GC and LC on macroscopic score of colitis. Abbreviations: Dia (diarrhea), Ulc. (ulcers), Ery. (erythema), Th. (thickness), Ad. (adhesion).
**Figure 6****:** Effect of treatment with different ratios of combination of GC and LC on serum IFNγ levels of TNBS induced colitis mice.
**Figure 7****:** Effect of treatment with different ratios of combination of GC and LC on IFN γ/IL4 ratio of TNBS induced colitis mice.
**Figure 8****:** Treatment with different ratios of combination of GC and LC resulted in increase in liver CD8 and NKT and demonstrates the beneficial effect of the tested glycolipids, in TNBS induced colitis mice. Abbreviations: liv. (liver).
**Figure 9****:** Effect of treatment with different ratios of combination of GC and LC on intrahepatic CD8+ T lymphoycte trapping (spleen/liver CD4/CD8 ratio) of TNBS induced colitis mice. Abbreviations: liv. (liver), sp. (spleen).
**Figure 10****:** Effect of glycolipids on tumor development (%) using HCC mice model.
**Figure 11****:** Effect of glycolipids on maximal tumor volume (mm³) using HCC mice model.
**Figure 12****:** Effect of glycolipids on tumor progression (% change from maximal volume) using HCC mice model.
**Figure 13****:** Effect of glycolipids on hepatic/splenic NKT lymphocyte ratio using HCC mice model.
**Figure 14****:** Effect of glycolipids on intrahepatic CD8+ T lymphoycte trapping (spleen/liver CD4/CD8 ratio) using HCC mice model.
**Figure 15****:** Effect of glycolipids on STAT 1, STAT 4 and STAT 6 expression (ODxmm²) using HCC mice model.
**Figure 16A-16C****:** Effect of different β-glycolipids on splenic and intrahepatic N KT lymphocytes using the ConA induced hepatitis model.

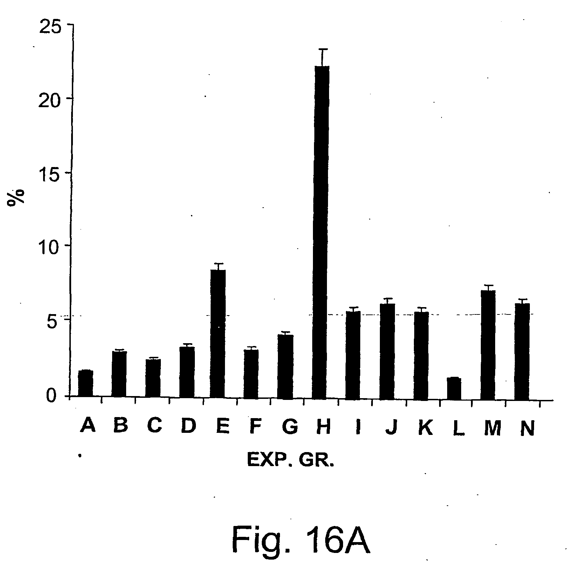

**Fig. 16A****:** intrahepatic N KT cells.

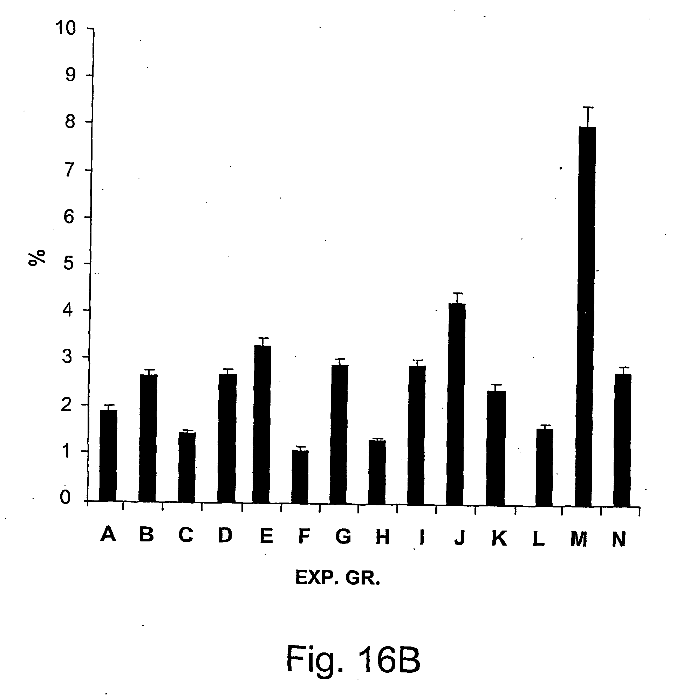

**Fig. 16B****:** spleen NK T cells.

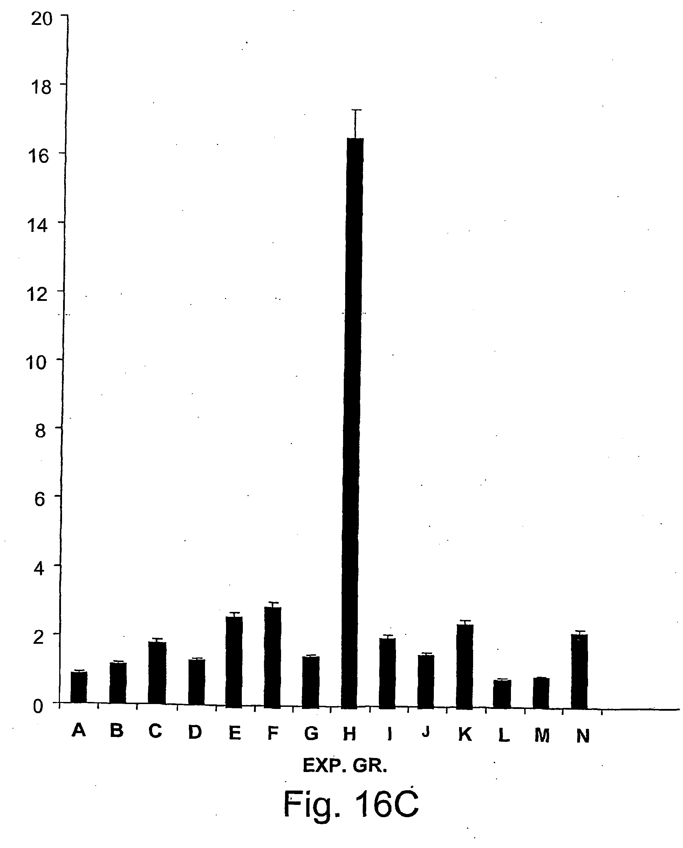

**Fig. 16C****:** liver/spleen NK T cell ratio. Abreviations: Exp. (experimental), gr. (groups).

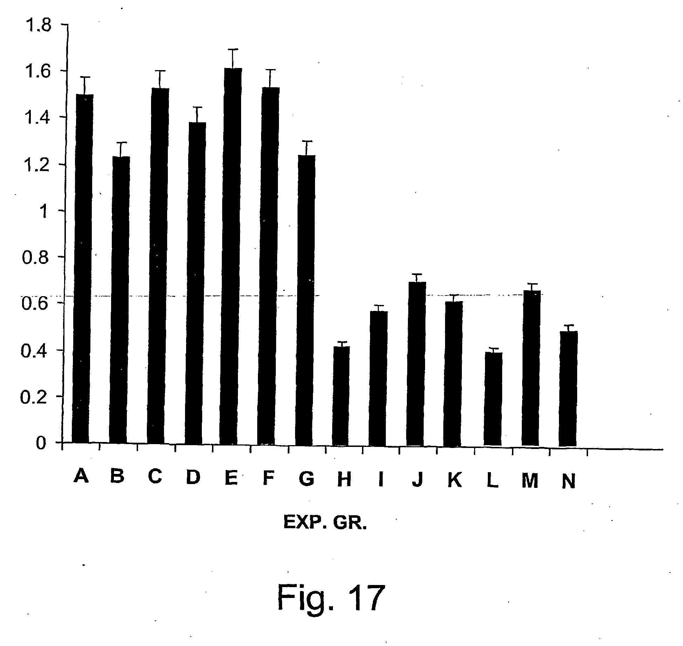

**Figure 17****:** Effect of β-glycolipids on splenic and intrahepatic CD4 and CD8 lymphocytes, using the ConA induced hepatitis model. Abreviations: Exp. (experimental), gr. (groups).

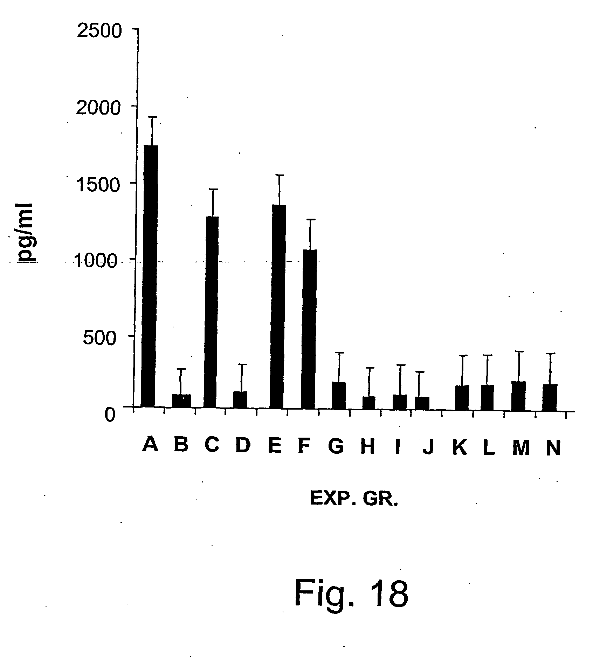

**Figure 18****:** Effect of β-glycolipids on serum cytokine levels (IFNγ) using the ConA induced hepatitis model. Abreviations: Exp. (experimental), gr. (groups).

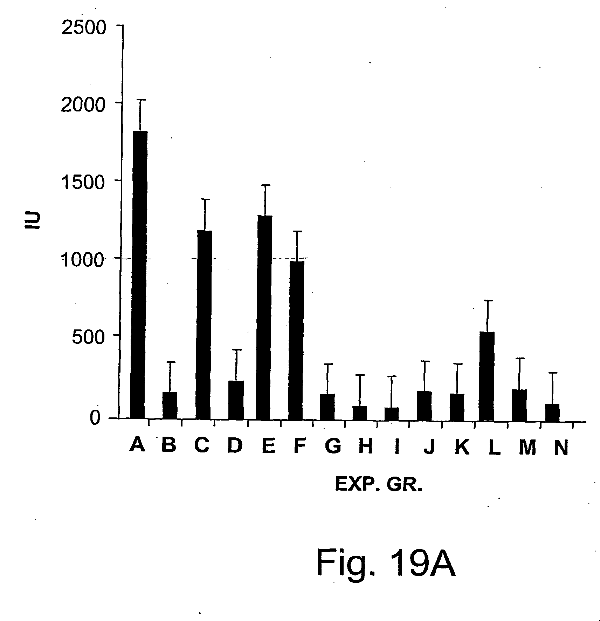

**Figure 19A-19B****:** Effect of different β-glycolipids on liver damage, using the ConA induced hepatitis model.
**Fig. 19A****:** serum AST levels.

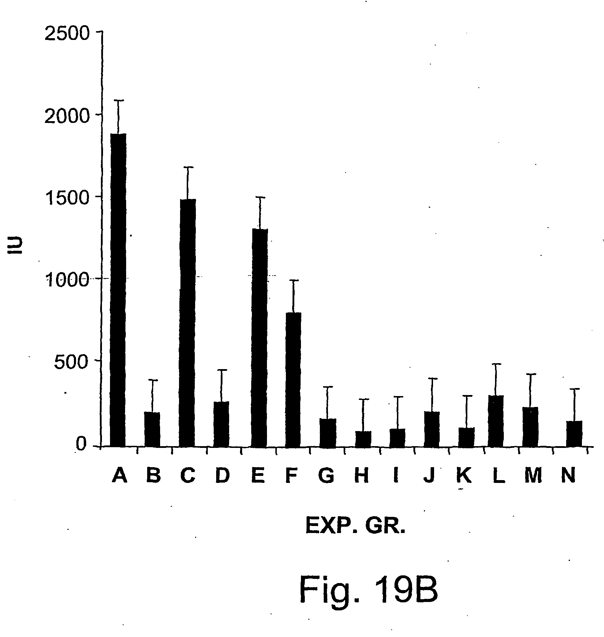

**Fig. 19B****:** serum ALT levels. Abreviations: Exp. (experimental), gr. (groups).

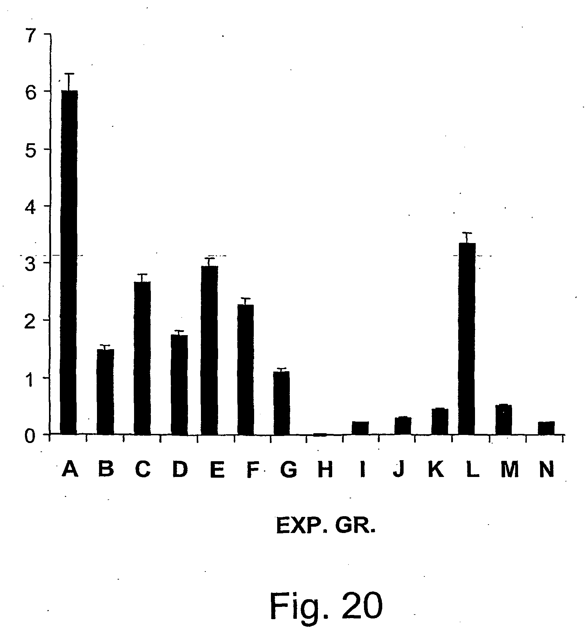

**Figure 20****:** Effect of β-glycolipids, on liver histology, using the ConA induced hepatitis model. Abreviations: Exp. (experimental), gr. (groups).

### Detailed Description of the Invention

As a first aspect, the present invention relates to a process for the modulation of the Th1/Th2 cell balance toward anti-inflammatory cytokine producing cells, in a subject suffering from an immune related disorder. This process comprises the step of increasing the intracellular, extra-cellular or serum level of a naturally occurring β-glycolipid in a subject in need thereof. It should be noted that the modulation may be mediated by at least one component of said subject immune system. According to this embodiment, increasing the intracellular, extra-cellular or serum level of a naturally occurring β-glycolipid in said subject may be performed by: (I) administering to the treated subject an effective amount of any one of: a mixture of at least two β-glycolipids or (II) exposing at least one component of said subject immune system to an effective amount of any one of: a mixture of at least two β-glycolipids,

A substance which increases the intracellular, extracellular or serum level of a naturally occurring β-glycolipid, may increase the rate of production of said β-glycolipid in said subject, or decrease the rate of degradation or turnover of said β-glycolipid in said subject.

, The mixture of β-glycolipids used by the process of the invention may comprise the two β-glycolipids at a quantitative ratio between 1:1 to 1:1000. It should be appreciated that any quantitative ratio may be used. As a non-limiting example, a quantitative ratio used may be: 1:1, 1:2, 1:3, 1:4, 1:5, 1:6, 1:7, 1:8, 1:9, 1:10, 1:20, 1:30, 1:40, 1:50, 1:60, 1:70, 1:80, 1:90, 1:100, 1:200, 1:300, 1:400, 1500, 1:750, 1:1000. It should be further noted that where the mixture of the invention comprises more than two glycolipids, the quantitative ratio used may be for example, 1:1:1, 1:2:3, 1:10:100, 1:10:100:1000 etc.

According to a specifically preferred embodiment, a mixture of preferred β-glycolipids used by the process of the invention comprises β-lactosyl-ceramide and at least one other β-glycolipid at a quantitative ratio between 1:1 to 1:1000. More preferably, such mixture comprises β-glucosylceramide (GC) and β-lactosyl-ceramide (LC) at a quantitative ratio between 1:1 to 1:1000. Most preferably, the mixture used by the process of the invention may comprise β-glucosylceramide (GC) and β-lactosyl-ceramide (LC) at a quantitative ratio of any one of 1:10 and 1:100, preferably, 1:10.

As shown by the following examples, different combinations of different ratios at different concentrations of GC and LC were examined using different model systems. For example, the TNBS induced colitis mouse model. As shown by Example 4, oral daily mixture of is µg GC + 150 µg LC (1:10) and 1.5µg GC + 150 µg LC (1:100), showed the best anti-inflammatory effects. Based on these results, a daily amount of such preferred mixtures, may contain between about 0.01 to 50, preferably, 0.5 to 5 mg per kg of body weight of β-glucosylceramide and between about 0.1 to 500, preferably, 5 to 50 mg per kg of body weight of β-lactosyl-ceramide at a quantitative ratio of 1:10 of 1:100, preferably of 1:10.

According to a particular embodiment, the mixture used by the process of the invention may comprises 0.75 mg per kg of body weight β-glucosylceramide and 7.5 mg per kg of body weight β-lactosyl-ceramide.

It should be appreciated that these preferred amounts of active ingredients are specific for a specific immune-related disorder, the colitis. Appropriate concentrations for any other immune-related disorders should be determined by the treating physician.

As indicated herein before, the process of modulation of the Th1/Th2 cell balance toward anti-inflammatory cytokine may be mediated by at least one component of the subject immune system. According to a preferred embodiment, such component may be selected from the group consisting of cellular immune reaction elements, humoral immune reaction elements and cytokines. Preferably, such component may be a cellular immune reaction element.

It should be noted that the process of the invention, in addition to being mediated by components of the immune-system of the treated subjects, may also be performed using a cellular component which was pre-exposed to glycolipids.

According to a particular embodiment, the cellular immune reaction element may be a population of NK T cells. NK T cells can be obtained from bone marrow, liver, spleen, or uterus, but can also be obtained from the peripheral blood, by cytopheresis methods.

Thus, according to a specifically preferred embodiment, increasing the intracellular, extra-cellular or serum level of a naturally occurring β-glycolipid by the process of the invention, may be performed by exposing at least one component of said subject immune system, preferably, NK T cells to an effective amount of any one of a β-glycolipid, a mixture of at least two β-glycolipids, a substance which increases the intracellular, extracellular or serum level of a naturally occurring β-glycolipid and any combination of the above.

According to this specifically preferred embodiment, the process of the invention is performed by the steps of: (a) obtaining NK T cells from said subject, or from a non autologous subject; (b) *ex vivo* educating the NK T cells obtained in step (a) such that the resulting educated NK T cells have the capability of modulating the Th1/Th2 cell balance toward anti-inflammatory cytokine producing cells; and (c) re-introducing to the treated subject the educated NK T cells obtained in step (b) which are capable of modulating the Th1/Th2 cell balance toward anti-inflammatory cytokine producing cells. This modulation results in an increase in the quantitative ratio between any one of IL4 and IL10 to IFNγ.

More particularly, *ex vivo* educating the NK T of step (b) may be performed by culturing the NK T cells in the presence of any one of: (a) a β-glycolipid, a mixture of at least two β-glycolipids, a substance which increases the intracellular, extracellular or serum level of a naturally occurring β-glycolipid, and any combination thereof; (b) antigens associated with said immune-related disorder or any combination thereof; (c) at least one of liver-associated cells of tolerized or non-tolerized subjects suffering from said immune-related disorder or of said subject; (d) at least one of cytokines, adhesion molecules or any combination thereof; (e) antigen presenting cells; and (f) a combination of any of (a), (b), (c), (d) and (e).

According to one embodiment, the NK T cell may be exposed to antigens associated with said immune-related disorder to be treated. These antigens may be for example, any one of allogeneic antigens obtained from a donor subject suffering from said immune-related disorder, xenogenic antigens, syngeneic antigens, autologous antigens, non-autologous antigens and recombinantly prepared antigens and any combinations thereof. These antigens can be native or non-native with regards to the subject. They can be natural or synthetic, modified or unmodified, whole or fragments thereof. Fragments can be derived from synthesis as fragments or by digestion or other means of modification to create fragments from larger entities. Such antigen or antigens comprise but are not limited to proteins, glycoproteins, enzymes, antibodies, histocompatibility determinants, ligands, receptors, hormones, cytokines, cell membranes, cell components, viruses, viral components, viral vectors, non-viral vectors, whole cells, tissues or organs. The antigen can consist of single molecules or mixtures of diverse individual molecules. The antigen can present itself within the context of viral surface, cellular surface, membrane, matrix, or complex or conjugated with a receptor, ligand, antibody or any other binding partner.

Polymerization and degradation, fractionation and chemical modification are all capable of altering the properties of a particular antigen in terms of potential immune responses. These small segments, fragments or epitopes can either be isolated or synthesized.

The method of the present invention further encompasses recombinantly prepared antigens. Preparation of recombinant antigens involves the use of general molecular biology techniques that are well known in the art. Such techniques include for example, cloning of a desired antigen to a suitable expression vector.

The liver was shown to play a role in T cell differentiation. CD3-CD4⁺/CD8⁺TCRβ cells and CD3-4-TCRβ⁺ cells can be generated from CD4-8-TCRβ athymic nude bone marrow cells by culture with liver parenchymal cells [Mabuchi, A., et al., J. Leukocyte Biology, 63:575-583 (1998)]. Therefore, in another particular embodiment, the *ex vivo* education of the NK T cells may be performed by culturing these cells in the presence of liver-associated cells. These cells may be for example Kupffer cells, Stellate cells, liver endothelial cells liver associated stem cells or any other liver-related lymphocytes.

Co-culturing of the NK T cells in the presence of peripheral lymphocytes from tolerized or non-tolerized patients suffering from the same immune-related disorder or from the treated subject, is also contemplated in the present invention. In order to obtain lymphocytes from a subject, particularly human subject, blood is drawn from the patient by cytopheresis, a procedure by which a large number of white cells are obtained, while other blood components are being simultaneously transferred back to the subject.

According to another embodiment, the NK T cell may be exposed to an antigen presenting cell that may be a dendritic cell.

In another particular embodiment, the *ex-vivo* education of the NK T cells may be performed by culturing the cells in the presence of cytokines such as IL4, IL10, TGFβ, IFNγ, IL12 and IL15, or in the presence of adhesion molecules such as Integrins, Selectin and ICAM.

In a specifically preferred embodiment, the NK T cell that has been *ex vivo* educated as described above may be re-introduced to the treated subject. This can be carried out by a process that has been termed adoptive transfer. The particular educated NK T cells used for the transfer may preferably originate from the subject (autologous transfer). A syngeneic or non-syngeneic donor (non-autologous transfer) is not excluded. The storage, growth or expansion of the transferred cells may have taken place *in vivo, ex vivo* or *in vitro.*

Cell therapy may be by injection, e.g., intravenously, or by any of the means described herein above. Neither the time nor the mode of administration is a limitation on the present invention. Cell therapy regimens may be readily adjusted taking into account such factors as the possible cytotoxicity of the educated cells, the stage of the disease and the condition of the patient, among other considerations known to those of skill in the art.

According to another embodiment, in addition to introducing to the treated subject *ex vivo* educated NK T cells, the process of the invention may further comprise the step of administering to said subject: (a) a β-glycolipid, a mixture of at least two β-glycolipids, a substance which increases the intracellular, extracellular or serum level of a naturally occurring β-glycolipid, and any combination thereof; (b) components, cells, tissues and/or organs derived from any one of allogeneic donors suffering from said immune-related disorder, xenogeneic sources and autologous sources, and immunologically functional equivalents, and combinations thereof; and (c) any combination of the above.

It is to be appreciated that the NK T cells may be educated *in vivo* as well, via any of the methods described above, they can be modulated prior to or at any point of time following exposure to the β-glycolipids, antigens or any other component described.

According to a specifically preferred embodiment, modulation of the Th1/Th2 cell balance toward anti-inflammatory cytokine producing cells by the process of the invention, may be performed by administering an effective amount of any one of: a β-glycolipid, a mixture of at least two β-glycolipids, a substance which increases the intracellular, extracellular or serum level of a naturally occurring β-glycolipid and any combination of the above. According to this embodiment, the administering step comprises oral, intravenous, intramuscular, subcutaneous, intraperitoneal, perenteral, transdermal, intravaginal, intranasal, mucosal, sublingual, topical, rectal or subcutaneous administration, or any combination thereof.

In yet another embodiment, the autoimmune disease may be any one of rheumatoid arthritis, diabetes, asthma, acute and chronic graft versus host disease, systemic lupus erythmatosus, scleroderma, multiple sclerosis, non alcoholic fatty liver disease, hyperlipidemia, atherosclerosis, the metabolic syndrome or any of the diseases comprising the same, obesity, inflammatory bowel disease and immune mediated hepatitis.

In a second aspect, the invention relates to a method for the treatment of immune-related disorder in a mammalian subject in need thereof. According to one embodiment, the method of treatment comprises the step of administering to said subject an effective amount of a mixture of at least two naturally occurring β-glycolipids;

The β-glycolipid used by the method of the invention is selected from the group consisting of a a glucosylceramid, a lactosyl-ceramide,

In yet another preferred embodiment, a mixture of β-glycolipids used by the method of the invention may comprise at least two β-glycolipids at a quantitative ratio between 1:1 to 1:1000. It should be appreciated that any quantitative ratio may be used. For example: 1:2, 1:50, 1:200, 1:350.

According to a specifically preferred embodiment, a mixture of preferred β-glycolipids used by the method of the invention may comprise β-lactosyl-ceramide and at least one other β-glycolipid at a quantitative ratio between 1:1 to 1:1000. More preferably, such mixture comprises β-glucosylceramide (GC) and β-lactosyl-ceramide (LC) at a quantitative ratio between 1:1 to 1:1000. Most preferably, the mixture used by the method of the invention may comprise β-glucosylceramide (GC) and β-lactosyl-ceramide (LC) at a quantitative ratio of any one of 1:10 and 1:100, preferably, 1:10.

Different combinations of different ratios at different concentrations of GC and LC may be used for different immune-related disorders. A daily dose of the active ingredients in a preferred mixture, may contain between about 0.01 to 50, preferably, 0.5 to 5 mg per kg of body weight of β-glucosylceramide (GC) and between about 0.1 to 500, preferably, 5 to 50 mg per kg of body weight of β-lactosyl-ceramide (LC) at a quantitative ratio of 1:10 of 1:100, preferably of 1:10.

According to a particular embodiment, the mixture used by the method of the invention may comprise 0.75 mg per kg of body weight β-glucosylceramide and 7.5 mg per kg of body weight β-lactosyl-ceramide.

It should be appreciated that these preferred amounts of active ingredients are specific for a specific immune-related disorder, the colitis. Appropriate concentrations for any other immune-related disorders should be determined by the treating physician.

According to one preferred embodiment, the method of treatment may be based on exposing a component of the treated subject's immune system to the different β-glycolipids. According to a preferred embodiment, such component may be selected from the group consisting of cellular immune reaction elements, humoral immune reaction elements and cytokines. Preferably, such component may be a cellular immune reaction element.

According to a particular embodiment, the cellular immune reaction element may be a population of NK T cells.

More specifically, exposing NK T cells to an effective amount of the β-glycolipids of the invention may be performed by the steps of: (a) obtaining NK T cells from said subject, or from another subject; (b) *ex vivo* educating the NK T cells obtained in step (a) such that the resulting educated NK T cells have the capability of modulating the Th1/Th2 cell balance toward anti-inflammatory cytokine producing cells; and (c) re-introducing to said subject the educated NK T cells obtained in step (b) which are capable of modulating the Th1/Th2 cell balance toward anti-inflammatory cytokine producing cells. Such modulation results in an increase in the quantitative ratio between any one of IL4 and IL10 to IFNγ.

More particularly, *ex vivo* educating the NK T of step (b) may be performed by culturing said NK T cells in the presence of any one of: (a) a β-glycolipid, a mixture of at least two β-glycolipids, a substance which increases the intracellular, extracellular or serum level of a naturally occurring β-glycolipid, and any combination thereof; (b) antigens associated with said immune-related disorder or any combination thereof; (c) at least one of liver-associated cells of tolerized or non-tolerized subjects suffering from said immune-related disorder or of said subject; (d) at least one of cytokines, adhesion molecules or any combination thereof; (e) antigen presenting cells; and (f) a combination of any of (a), (b), (c), (d) and (e).

According to one embodiment, the NK T cell may be exposed to antigens associated with said immune-related disorder. Such antigens may be for example, any one of allogeneic antigens obtained from a donor subject suffering from said immune-related disorder, xenogenic antigens, syngeneic antigens, autologous antigens, non-autologous antigens and recombinantly prepared antigens and any combinations thereof. According to another embodiment, the NK T cell may be exposed to liver-associated cells which may be selected from the group consisting of Kupffer cells, Stellate cells, liver endothelial cells, liver-associated stem cells and any other liver-related lymphocytes.

In yet another embodiment, the NK T cell may be exposed to cytokines such as IL4, IL10, TGFβ, IFNγ, IL12, IL2, IL18 and IL15.

Still further, the NK T cell may be exposed to adhesion molecules selected from the group consisting of Integrins, Selectin and ICAM.

According to another embodiment, the NK T cell may be exposed to an antigen presenting cell that may be a dendritic cell.

In a specifically preferred embodiment, the educated NK T cells may be re-introduced to the treated subject by adoptive transfer.

According to another embodiment, in addition to introduction of *ex vivo* educated NK T cells, the method of the invention may further comprise the step of administering to the treated subject: (a) a β-glycolipid, a mixture of at least two β-glycolipids, a substance which increases the intracellular, extracellular or serum level of a naturally occurring β-glycolipid, and any combination thereof (b) components, cells, tissues and/or organs derived from any one of allogeneic donors suffering from said immune-related disorder, xenogeneic sources and autologous sources, and immunologically functional equivalents, and combinations thereof; and (c) any combination of the above.

According to another embodiment, the method of the invention comprises administering to the treated subject an effective amount of any one of: a β-glycolipid, a mixture of at least two β-glycolipids, a substance which increases the intracellular, extracellular or serum level of a naturally occurring β-glycolipid and any combination of the above. According to this embodiment, the administering step comprises oral, intravenous, intramuscular, subcutaneous, intraperitoneal, perenteral, transdermal, intravaginal, intranasal, mucosal, sublingual, topical, rectal or subcutaneous administration, or any combination thereof.

Therapeutic formulations may be administered in any conventional dosage formulation. Formulations typically comprise at least one active ingredient, as defined above, together with one or more acceptable carriers thereof.

Each carrier should be both pharmaceutically and physiologically acceptable in the sense of being compatible with the other ingredients and not injurious to the patient. Formulations include those suitable for oral, rectal, nasal, or parenteral (including subcutaneous, intramuscular, intravenous and intradermal) administration. The formulations may conveniently be presented in unit dosage form and may be prepared by any methods well known in the art of pharmacy. The nature, availability and sources, and the administration of all such compounds including the effective amounts necessary to produce desirable effects in a subject are well known in the art and need not be further described herein.

According to one preferred embodiment, the method of the invention is intended for the treatment of immune disorder such as autoimmune disease, malignant and non-malignant proliferative disorder, graft rejection pathology, inflammatory disease, genetic disease, bacterial infections, viral infections, fungal infections, or parasitic infections.

In another specifically preferred embodiment, the method of the invention is intended for the treatment of a malignancy. In cancerous situations, modulation of the NK T cells may be in the direction of inducing a pro-inflammatory response or in augmenting the anti-tumor associated antigens immunity. As used herein to describe the present invention, "cancer", "tumor" and "malignancy" all relate equivalently to a hyperplasia of a tissue or organ. If the tissue is a part of the lymphatic or immune systems, malignant cells may include non-solid tumors of circulating cells. Malignancies of other tissues or organs may produce solid tumors. In general, the methods and compositions of the present invention may be used in the treatment of non-solid and solid tumors.

In yet another embodiment, the autoimmune disease may be any one of rheumatoid arthritis, diabetes, asthma, acute and chronic graft versus host disease, systemic lupus erythmatosus, scleroderma, multiple sclerosis, non alcoholic fatty liver disease, hyperlipidemia, atherosclerosis, the metabolic syndrome or any of the diseases comprising the same, obesity, inflammatory bowel disease and immune mediated hepatitis.

According to a particular embodiment, the viral infection may be caused by any one of HBV, HCV or HIV.

According to a specifically preferred embodiment, wherein the treated subject is suffering from any of the diseases indicated above, a preferred result of the treatment by the method of the invention may be for example, an increase in glucose tolerance, reduction in liver fat content or change in cytokine responses, reduction of tumor mass, increase in survival and amelioration of disease symptoms. Such results are demonstrated by the following examples.

According to another specific embodiment, the process of the invention is particularly intended for the treatment of a subject suffering from inflammatory bowel disease, such as collitis.

According to another specific embodiment, the method of the invention is particularly intended for the treatment of a subject suffering from immune mediated, viral or chemical mediated hepatitis.

Although the methods of the invention are particularly intended for the treatment of immune-related disorders in humans, other mammals are included. By way of non-limiting examples, mammalian subjects include monkeys, equines, cattle, canines, felines, mice, rats and pigs.

In a third aspect, the invention relates to a therapeutic composition for the treatment of an immune-related disorder in a mammalian subject. According to one embodiment, the composition of the invention may comprise as an active ingredient: any one of: a mixture of at least two β-glycolipids; and an educated NKT cells pre-exposed to any one of a β-glycolipid, a mixture of at least two β-glycolipids, a substance which increases the intracellular, extracellular or serum level of a naturally occurring β-glycolipid, or any combination thereof; .

In another embodiment, the composition of the invention may optionally further comprising any one of: (a) antigens associated with said immune-related disorder; (b) at least one of liver-associated cells of tolerized or non-tolerized subjects suffering from said immune-related disorder or of said subject; (c) at least one of cytokines, adhesion molecules or any combination thereof; (d) antigen presenting cells; and (e) a combination of any of (a), (b), (c) and (d).

According to one embodiment, the substance which increases the intracellular, extracellular or serum level of a naturally occurring β-glycolipid; may be a substance which increases the rate of production of said β-glycolipid in said subject, or a substance which decreases the rate of degradation or turnover of said β-glycolipid in said subject.

According to another preferred embodiment the β-glycolipid comprised within the composition of the invention may be selected from the group consisting of a monosaccharide ceramide, a glucosylceramide, a galatosylceremide, a lactosyl-ceramide, a gal-gal-glucosyl-ceramide, GM2 ganglioside, GM3 ganglioside, globoside or any other β-glycolipid. Preferably, the β-glycolipid used by the process of the invention may be β-lactosyl-ceramide and any analogue or derivative thereof. Compositions comprising a β-glycolipid other than glucosylceramide are also within the scope of the invention.

In yet another preferred embodiment, a mixture of β-glycolipids used by the composition of the invention may comprise at least two β-glycolipids at a quantitative ratio between 1:1 to 1:1000. It should be appreciated that any quantitative ratio may be used.

According to a specifically preferred embodiment, a mixture of preferred β-glycolipids comprised within the composition of the invention may comprise β-lactosyl-ceramide and at least one other β-glycolipid at a quantitative ratio between 1:1 to 1:1000. Such mixture comprises β-glucosylceramide and β-lactosyl-ceramide at a quantitative ratio between 1:1 to 1:1000. Most preferably, the mixture used for the composition of the invention may comprise β-glucosylceramide (GC) and β-lactosyl-ceramide (LC) at a quantitative ratio of any one of 1:10 and 1:100, preferably, 1:10.

Different combinations of different ratios at different concentrations of GC and LC may be used for a composition useful for different immune-related disorders. A daily dose of the active ingredients in a preferred mixture, may contain between about 0.01 to 50, preferably, 0.5 to 5 mg per kg of body weight of β-glucosylceramide (GC) and between about 0.1 to 500, preferably, 5 to 50 mg per kg of body weight of β-lactosyl-ceramide (LC) at a quantitative ratio of 1:10 of 1:100, preferably of 1:10.

According to a particular embodiment, the mixture used for the composition of the invention may comprise 0.75 mg per kg of body weight β-glucosylceramide and 7.5 mg per kg of body weight β-lactosyl-ceramide.

It should be appreciated that these preferred amounts of active ingredients are preferred for a composition for the treatment of colitis. Appropriate concentrations for any other immune-related disorders should be determined by the treating physician.

According to another preferred embodiment, the therapeutic composition of the invention may comprise as an active ingredient, educated NK T cells capable of modulating the Th1/Th2 cell balance toward anti-inflammatory cytokine producing cells. More specifically, the educated NK T cells comprised within the composition of the invention were cultured in the presence of any one of: (a) a β-glycolipid, a mixture of at least two β-glycolipids, a substance which increases the intracellular, extracellular or serum level of a naturally occurring β-glycolipid, or any combination thereof; (b) antigens associated with said immune-related disorder; (c) at least one of liver-associated cells of tolerized or non-tolerized subjects suffering from said immune-related disorder or of said subject; (d) at least one of cytokines, adhesion molecules and any combination thereof (e) antigen presenting cells; and (f) a combination of any of (a), (b), (c), (d) and (e).

According to another preferred embodiment, the composition of the invention comprises ex vivo educated NKT cells. These cells were exposed to antigens associated with the immune-related disorder to be treated. Such antigens may for example, allogeneic antigens obtained from a donor subject suffering from said immune-related disorders, xenogenic antigens, syngeneic antigens, autologous antigens, non- autologous antigens and recombinantly prepared antigens and any combinations thereof.

According to one embodiment, the NK T cell may be exposed to antigens associated with said immune-related disorder. Such antigens may be for example, any one of allogeneic antigens obtained from a donor subject suffering from said immune-related disorder, xenogenic antigens, syngeneic antigens, autologous antigens, non-autologous antigens and recombinantly prepared antigens and any combinations thereof.

According to another embodiment, the NK T cell may be exposed to liver-associated cells which may be selected from the group consisting of Kupffer cells, Stellate cells, liver endothelial cells, liver-associated stem cells and any other liver-related lymphocytes.

In yet another embodiment, the NK T cell may be exposed to cytokines such as IL4, IL10, TGFβ, IFNγ, IL12, IL2, IL18 and IL15.

Still further, the NK T cell may be exposed to adhesion molecules selected from the group consisting of Integrins, Selectin and ICAM.

According to another embodiment, the NK T cell may be exposed to an antigen presenting cell that may be a dendritic cell.

According to one embodiment, the composition of the invention is intended for the treatment of an immune disorder such as an autoimmune disease, malignant and non-malignant proliferative disorder, graft rejection pathology, inflammatory disease, genetic disease, bacterial infections, viral infections, fungal infections, or parasitic infections.

In yet another embodiment, the autoimmune disease may be any one of rheumatoid arthritis, diabetes, asthma, acute and chronic graft versus host disease, systemic lupus erythmatosus, scleroderma, multiple sclerosis, non alcoholic fatty liver disease, hyperlipidemia, atherosclerosis, the metabolic syndrome or any of the diseases comprising the same, obesity, inflammatory bowel disease and immune mediated hepatitis.

According to another specific embodiment, the composition of the invention is particularly intended for the treatment of a subject suffering from inflammatory bowel disease.

According to another specific embodiment, the composition of the invention is particularly intended for the treatment of a subject suffering from immune mediated, viral or chemical mediated hepatitis.

According to a particular embodiment, the viral infection comprises HBV, HCV or HIV.

The pharmaceutical compositions of the invention generally comprise a buffering agent, an agent which adjusts the osmolarity thereof, and optionally, one or more pharmaceutically acceptable carriers, excipients and/or additives as known in the art. Supplementary active ingredients can also be incorporated into the compositions. The carrier can be solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants.

As used herein "pharmaceutically acceptable carrier" includes any and all solvents, dispersion media, coatings, antibacterial and antifungal agents and the like. The use of such media and agents for pharmaceutical active substances is well known in the art. Except as any conventional media or agent is incompatible with the active ingredient, its use in the therapeutic composition is contemplated.

In a further aspect the invention relates to the use of a therapeutically effective amount of any one of: (a) (b) a mixture of at least two β-glycolipids; in the preparation of a composition for the treatment of an immune-related disorder. According to a specific embodiment, the composition is as described by the invention.

The invention further provides a method for the preparation of a medicament for the treatment of an immune related disorder in a subject in need thereof. The method of the invention may comprise the following steps: (a) obtaining a component of the immune system of said subject from said subject, or from another subject; and (b) *ex vivo* exposing by culturing or incubating said component obtained in step (a) with an effective amount of any one of a β-glycolipid, a mixture of at least two β-glycolipids, a substance which increases the intracellular, extracellular or serum level of a naturally occurring β-glycolipid, or any combination thereof, such that the resulting component has the capability of modulating the Th1/Th2 cell balance toward anti-inflammatory cytokine producing cells.

According to one embodiment, the component of said subject immune system may be a cellular immune reaction element. More specifically, a population of NK T cells.

According to a specifically preferred embodiment, the method of the invention may be performed by the steps of: (a) obtaining NK T cells from said subject, or from another subject; and (b) *ex vivo* educating the NK T cells obtained in step (a) by culturing said NK T cells in the presence of any one of: (i) a β-glycolipid, a mixture of β-glycolipids, a substance which increases the intracellular, extracellular or serum level of a naturally occurring β-glycolipid, or any combination thereof; (ii) antigens associated with said immune-related disorder or any combination thereof; (iii) at least one of liver-associated cells of tolerized or non-tolerized subjects suffering from said immune-related disorder or of said subject; (iv) at least one of cytokines, adhesion molecules or any combination thereof; (v) antigen presenting cells; and (vi) a combination of any of (a), (b), (c), (d) and (e);

According to this preferred embodiment, the resulting educated NK T cells have the capability of modulating the Th1/Th2 cell balance toward anti-inflammatory cytokine producing cells.

The invention further provides a method for the preparation of a medicament for the treatment of an immune related disorder in a subject in need thereof comprising the steps of: (I) providing an immunomodulatory compound comprising any one of:
(a) a β-glycolipid; (b) a mixture of at least two β-glycolipids; (c) a substance which increases the intracellular, extracellular or serum level of a naturally occurring β-glycolipid; (d) educated NKT cells pre-exposed to any one of a β-glycolipid, a mixture of at least two β-glycolipids, a substance which increases the intracellular, extracellular or serum level of a naturally occurring β-glycolipid, or any combination thereof; and (e) any combinations of (a), (b), (c) and (d); and (II) admixing the immunomodulatory compound provided in step (a) with a pharmaceutically acceptable carrier.

In a further aspect, the invention relates to a composition for the modulation of the Th1/Th2 cell balance toward the Th2 anti-inflammatory cytokine producing cells. The composition of the invention comprising as an active ingredient an immunomodulatory effective amount of any one of: (a) a β-glycolipid; (b) a mixture of at least two β-glycolipids; (c) a substance which increases the intracellular, extracellular or serum level of a naturally occurring β-glycolipid; (d) educated NKT cells pre-exposed to any one of a β-glycolipid, a mixture of at least two β-glycolipids, a substance which increases the intracellular, extracellular or serum level of a naturally occurring β-glycolipid, or any combination thereof; and (e) any combinations of (a), (b), (c) and (d).

According to one embodiment, the immunomodulatory composition of the invention may optionally further comprises any one of: (a) antigens associated with said immune-related disorder; (b) at least one of liver-associated cells of tolerized or non-tolerized subjects suffering from said immune-related disorder or of said subject; (c) at least one of cytokines, adhesion molecules or any combination thereof; (d) antigen presenting cells; and (e) a combination of any of (a), (b), (c) and (d).

The said β-glycolipid is selected from the group consisting of a glucosylceramide, a lactosyl-ceramide,

According to another preferred embodiment the β-glycolipid comprised within the composition of the invention may be selected from the group consisting of a glucosylceramide, and a lactosyl-ceramide,

In yet another preferred embodiment, the mixture of β-glycolipids used by the composition of the invention may comprise at least two β-glycolipids at a quantitative ratio between 1:1 to 1:1000. It should be appreciated that any quantitative ratio may be used.

According to a specifically preferred embodiment, a mixture of preferred β-glycolipids comprised within the composition of the invention may comprise β-lactosyl-ceramide and at least one other β-glycolipid at a quantitative ratio between 1:1 to 1:1000. More preferably, such mixture comprises β-glucosylceramide and β-lactosyl-ceramide at a quantitative ratio between 1:1 to 1:1000. Most preferably, the mixture used for the composition of the invention may comprise β-glucosylceramide (GC) and β-lactosyl-ceramide (LC) at a quantitative ratio of any one of 1:10 and 1:100, preferably, 1:10.

Different combinations of different ratios at different concentrations of GC and LC may be used for a composition useful for different immune-related disorders. A daily dose of the active ingredients in a preferred mixture, may contain between about 0.01 to 50, preferably, 0.5 to 5 mg per kg of body weight of β-glucosylceramide (GC) and between about 0.1 to 500, preferably, 5 to 50 mg per kg of body weight of β-lactosyl-ceramide (LC) at a quantitative ratio of 1:10 of 1:100, preferably of 1:10.

According to a particular embodiment, the mixture used for the composition of the invention may comprise 0.75 mg per kg of body weight β-glucosylceramide and 7.5 mg per kg of body weight β-lactosyl-ceramide.

The invention further provides the use of the immuno-modulating composition of the invention, as a supporting medicament for the treatment of immune-related disorder.

Still futher, the invention provides for the use of the immuno-modulating composition according to the invention as an adjuvant for a vaccine against an immune-related

Disclosed and described, it is to be understood that this invention is not limited to the particular examples, methods steps, and compositions disclosed herein as such methods steps and compositions may vary somewhat. It is also to be understood that the terminology used herein is used for the purpose of describing particular embodiments only and not intended to be limiting since the scope of the present invention will be limited only by the appended claims and equivalents thereof.

It must be noted that, as used in this specification and the appended claims, the singular forms "a", "an" and "the" include plural referents unless the content clearly dictates otherwise.

Throughout this specification and the Examples and claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The following examples are representative of techniques employed by the inventors in carrying out aspects of the present invention. It should be appreciated that while these techniques are exemplary of preferred embodiments for the practice of the invention, those of skill in the art, in light of the present disclosure, will recognize that numerous modifications can be made without departing from the spirit and intended scope of the invention.

### Examples

### Experimental procedures

### Animals

*Normal inbred 2 to 4 month old C57B1 male mice were obtained from Jackson Laboratories, USA.
* Normal inbred 2 to 4 month old Balb/C male mice were obtained from Jackson Laboratories, USA.
*Ten-week-old male leptin-deficient C57BL/6J mice and lean C57BL/6 mice were purchased from Harlan laboratories.
*Eight week old male C57/bl mice were obtained from Jackson Laboratories (Bar Harbor, ME, USA).

All animals were maintained in the Animal Core of the Hadassah-Hebrew University Medical School. Mice were administered standard laboratory chow and water *ad libitum,* and kept in 12-hour light/dark cycles. Animal experiments were carried out according to the guidelines of the Hebrew University-Hadassah Institutional Committee for Care and Use of Laboratory Animals, and with the committee's approval.

### β-glycolipids

The following β-glycolipids were used: β-glucosylceramide (also indicated as GluC or GC), β-lactosyl-ceramide (also indicated as LacC or LC), and β-galactosyl-ceramide (GalC), iGb3 and ceramide

### Induction of Colitis

2,4,6-trinitrobenzene sulfonic acid (TNBS) - colitis was induced by intracolonic installation of TNBS, 1 mg/mouse, dissolved in 100 ml of 50% ethanol as described. [Collins, C., et al., Eur. J. Immunol. 26:3114-3118 (1996)].

### Clinical Assessment of Colitis

Diarrhea was followed daily throughout the study.

### Macroscopic Score of Colitis

Colitis assessment was performed 14 days following colitis induction using standard parameters [Madsen, K.L., et al., Gastroenterology 113:151-159 (1997); Trop, S., et al., Hepatology 27:746-755 (1999)].

Four macroscopic parameters were determined, namely: degree of colonic ulcerations, intestinal and peritoneal adhesions, wall thickness and degree of mucosal edema. Each parameter was graded on a scale from 0 (completely normal) to 4 (most severe) by two experienced blinded examiners.

### Grading of Histological Lesions

For histological evaluation of inflammation, distal colonic tissue (last 10 cm) was removed and fixed in 10% formaldehyde. Five paraffin sections from each mouse were then stained with hematoxyllin-eosin by using standard techniques. The degree of inflammation on microscopic cross sections of the colon was graded semiquantitatively from 0 to 4 [Madsen *et al.,* (1997) *ibid.;* Trop et al., Hepatology 27:746-755 (1999)]. Grade 0: normal with no signs of inflammation; Grade 1: very low level of leukocyte infiltration; Grade 2: low level of leukocyte infiltration; and Grade 3: high level of infiltration With high vascular density, and bowel wall thickening; Grade 4: transmural infiltrates with loss of goblet cells, high vascular density, wall thickening, and disruption of normal bowel architecture. The grading was performed by two experienced blinded examiners.

### Splenic and Hepatic Lymphocyte Isolation

Splenocytes were isolated and red blood cells removed as previously described [Vicari, A.P., et al., Immunology Today 17(2):71 (1996)]. Intrahepatic lymphocytes were isolated from all groups of mice at the end of the study, as previously described, with some modifications [Vicari *et al.,* (1996) *ibid.;* Bleicher, P.A., et al., Science 250:679-682 (1990)]. The inferior *vena cava* was cut above the diaphragm and the liver was flushed with 5 ml of cold PBS until it became pale. The connective tissue and the gall bladder were removed, and livers were placed in a 10-ml dish in cold sterile PBS. Livers and spleens were crushed through a stainless mesh

(size 60, Sigma Chemical Co., St. Louis MO). Cell suspension was placed in a 50 ml tube for 3 minutes and washed twice in cold PBS (1,250xrpm for 10 minutes), and debris was removed. Cells were re-suspended in PBS, cell suspension was placed through a nylon mesh presoaked in PBS, and unbound cells were collected. Cells were washed twice in 45 ml PBS (1,250xrpm in room temperature). For liver and spleen lymphocyte isolation 20 ml of histopague 1077 (Sigma Diagnostics, St. Louis, MO) were slowly placed underneath the cells suspended in 7 ml of PBS, in a 50-ml tube. The tube was centrifuged at 1,640 rpm for 15 minutes at room temperature. Cells at the interface were collected, diluted in a 50-ml tube, and washed twice with ice-cold PBS (1,250 rpm for 10 minutes). Approximately 1x10⁶ cells/mouse liver were recovered. The viability by trypan blue staining was more than 95%. Both splenocytes and liver-associated lymphocytes were isolated from all animals in all experimental groups.

### Induction of Con A Hepatitis

Con A (Sigma) was dissolved in pyrogen-free PBS and injected into the tail vein at a dose of 500 µg/mouse (approximately 15 mg/kg).

### FACS of Intrahepatic and Intrasplenic Lymphocytes for NKT, CD4 and CD8 Markers

Immediately following lymphocyte isolation, triplicates of 2-5x10⁴ cells/500µl PBS were put into Falcon 2052 tubes incubated with 4 ml of 1% BSA for 10 minutes, and centrifuged at 1400 rpm for 5 minutes. Analysis of lymphocyte subpopulations was performed using anti-NK1.1, anti-CD3, anti-CD4 and anti CD-8 antibodies. Cells were washed twice in 1% BSA, and kept at 4°C until reading. For the control group, only 5µl of 1% BSA was added. Analytical cell sorting was performed on 1x10⁴ cells from each group with a fluorescence-activated cell sorter (FACSTAR plus, Becton Dickinson). Only live cells were counted, and background fluorescence from non-antibody-treated lymphocytes was deducted from the levels obtained. Gates were set on forward- and side-scatters to exclude dead cells and red blood cells. The data were analyzed with Consort 30 two-color contour plot program (Becton Dickinson, Oxnard, CA), or the CELLQuest program. ,

### Measurement of Cytokine Levels

Blood was drawn from mice in all groups and centrifuged at 14,000 rpm. Serum IFNγ, IL2, IL4, IL10 and IL-12 levels were measured by "sandwich" ELISA using Genzyme Diagnostics kits (Genzyme Diagnostics, MA).

### Glucose Tolerance Test

Glucose tolerance was assessed by oral administration of glucose (1 gram per kilogram body weight). Blood drawn from the tail was measured for glucose at 0', 15', 30', 60', 90', 120' and 180'. Glucose levels were measured with Elite glucose test strips and a glucometer.

### Hepatic MRI Measurement of Fat Content

Hepatic fat content was measured using a double-echo chemical shift gradient-echo magnetic resonance imaging (MRI) sequence that provides in-phase and opposed-phase images in a single acquisition for assessment/quantification of fat in mouse liver. The T1-weighted opposed-phase MR imaging technique is sensitive for detection of relatively small amounts of tissue fat. TRI images were performed with a 1.5-T system (Sigma LX; GE, Milwaukee, USA). Double-echo MR imaging was performed with a repetition time (TR) of 125 msec, double echo times (TEs) of 4 and 6.5 msec, and a flip angle of 80°. Imaging parameters included section thickness of 3mm, 13-cm field of view, 256* 160 matrix, and one signal acquired, with use of a knee coil. Transverse (axial) and coronal images were acquired at the level of the liver with a 3mm section thickness and no intersection gap. Quantitative assessment of signal intensity (SI) measurements of SI changes between in-phase and opposed-phase images was computed as described in previous reports [Mitchell, D.G. et al., Invest. Radiol 26:1041-1052 (1991); Tomohiro, N. et al., Radiology 218:642-646 (2001)]. The SI index was calculated as follows: SI index = (Slip-Siₒₚ)/SIip, where SIᵢₚ is SI on in-phase images and SIₒₚ is SI on opposed-phase images. The SI index reflects the fraction of SI loss on opposed phase images compared with the SI on in-phase images.

### Histological Examination

Hematoxyllin/eosin staining of paraffin-embedded liver sections was performed. Sections were examined by two experienced pathologists that were blinded to the experiment conditions.

### Example 1

### Use of β-glycolipids for treatment of immune mediated colitis

To determine the clinical and immunological effect of administration of β-glycolipids such as β-glucosylceramide (GluC), β-lactosyl-ceramide (LacC), and β-galactosyl-ceramide (GalC) and of ceramide on a murine model of experimental colitis, nine groups of C57B1 mice, consisting of 10 mice each, were studied. As summarized in Table 1, colitis was induced by intracolonic installation of trinitrobenzenesulfonic acid (TNBS) on day 1 and 5 in groups A - E. Group A mice were fed regular chow diet. Group B - E mice received oral (PO) 15µg daily of GluC, LacC, GalC and ceramide, respectively. Groups F - I mice were not treated with TNBS, but received oral (PO) 15µg daily of GluC, LacC, GalC and ceramide, respectively, and served as control groups.

Mice were followed for macroscopic and microscopic colitis scores. The immunemodulatory effect of GC was determined by FACS analysis of intrahepatic and intrasplenic lymphocytes for NKT, CD4 and CD8 markers, and by measurement of serum IFNγ, IL2, IL12, IL4 and IL10 cytokine levels.

**Table 1**

| Group: | Treatment: |
|---|---|
| A | TNBS |
| B | TNBS with OP 15 µg β-GluC |
| C | TNBS with OP 15 µg -LacC |
| D | TNBS with OP 15 µg β-GalC |
| E | TNBS with OP 15 µg ceramide |
| F | OP 15 µg β-GluC |
| G | OP 15 µg β-LacC |
| H | OP 15 µg β-GalC |
| I | OP 15 µg ceramide |

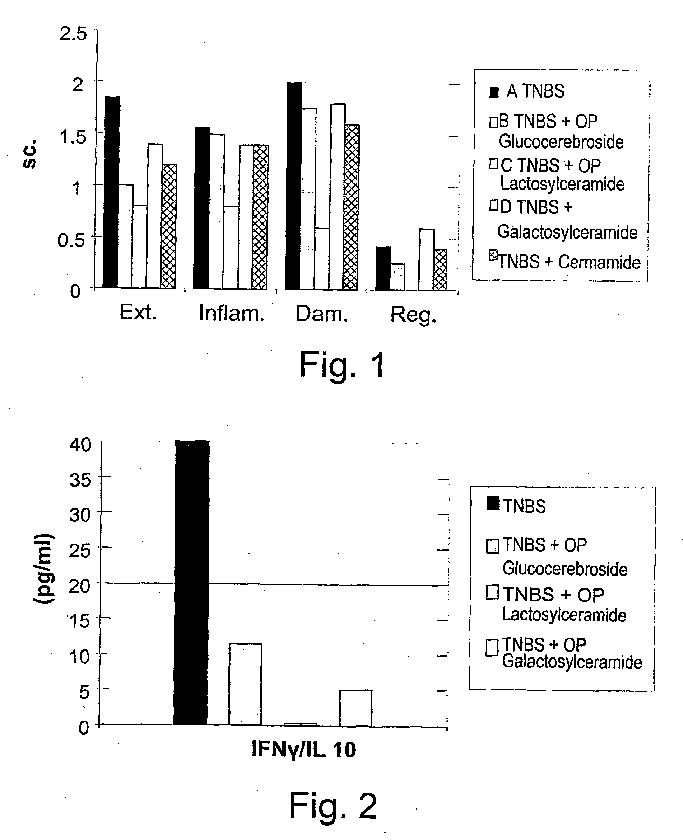

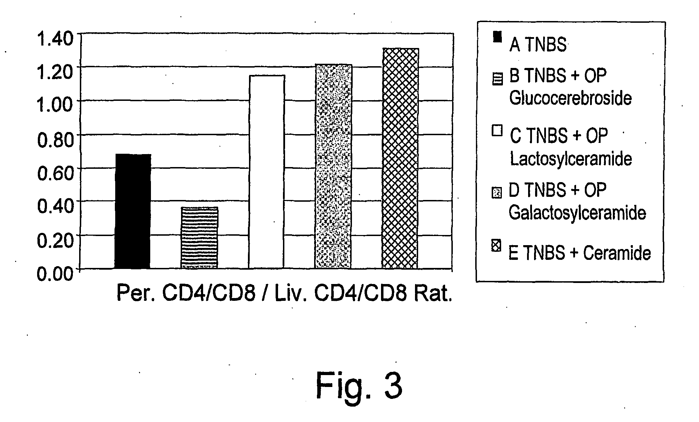

As shown in Figure 1, administration of β-glycolipids resulted in alleviation of colitis, most marked for LacC, with improvement of the microscopic colitis scores as compared with controls. Alleviation of colitis by β-glycolipid treatment was associated with a significant increase of intrahepatic CD8+ T cell trapping (Figure 3). The beneficial effect of β-glycolipids on TNBS colitis was associated with an increase in the number of intrahepatic NKT cells. As clearly shown in Figure 2, administration of beta glycolipids led to a decreased serum IFNγ level and decreased IFNγ/IL-10 ratio. As was demonstrated by the control groups (F-I), administration of different β-glycolipids to naive mice did not adversely affect functional status, weight, liver and colon pathology.

The data clearly indicate that β-glycolipids, alleviate experimental colitis in a murine model. This alleviation was accompanied by increased intrahepatic NKT lymphocytes, increased intrahepatic CD8 T lymphocyte trapping, and a shift toward a Th2 cytokine profile (as indicated by the reduced IFNγ/IL-10 ratio). The extent of this effect varies according to the different glycolipid used. Lactosyl-ceramide (LC) was found to be the most potent in this respect.

### Example 2

### Synergistic effect for mixtures of β-glycolipids in the treatment of immune mediated colitis

To determine the possible immuno-modulation effect of a mixture of different β-glycolipids, the effect of a mixture of β-Glucocerebroside (GC) and β-Lactosyl-ceramide (LC) was tested using a murine model of colitis.

As summarized in Table 2, five groups of mice were studied, each consisting of 10 mice. Colitis was induced by intracolonic installation of trinitrobenzenesulfonic acid (TNBS) on day 1 and 5 in groups A, B and D. Group A mice were fed regular chow diet. Group B mice received oral (PO) 15µg daily of a mixture of β-GluC (GC) and β-LacC (LC), group D received only β-LacC. Groups C and E mice were not treated with TNBS, but received oral (PO) 15µg daily of a mixture of β-GluC and β-LacC or only β-LacC, respectively, and served as control groups.

Mice were followed for macroscopic and microscopic colitis scores, as well as for survival and functional status and weight.

**Table 2**

| Group: | Treatment: |
|---|---|
| A | TNBS |
| B | TNBS with 15 µg β-GluC + 15 µg β-LacC |
| C | 15 mg β-GluC + 15 µg β-LacC |
| D | TNBS with 15 µg β-LacC |
| E | 15 µg β-LacC |

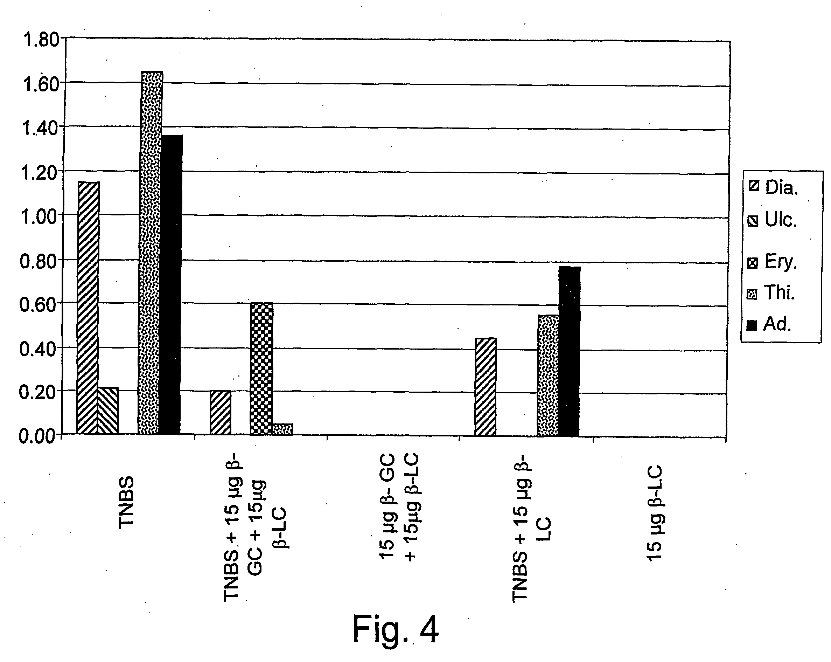

A clear synergistic effect of the β-GluC+β-LacC mixture is demonstrated by Figure 4. As shown by this figure, administration of a mixture of both β-GlucC and β-LacC, led to significant amelioration of TNBS induced colitis, as indicated by the reduction in diarrhea, ulcers erythema, thickness and adhesions. This effect was much more significant in the β-GluC+β-LacC mixture group, when compared to the effect of β-LacC alone. As indicated by Table 3, the synergistic effect of the β-GluC+β-LacC mixture was further demonstrated by improved functional status, weight and 40% increase in survival.

**Table 3**

| **Group** | **Activity** | **Weight** |
|---|---|---|
| TNBS | Lethargic | Weight loss |
| TNBS with 15µg β-GC SOY + 15 | Normal | In 80% no change |
| µg β-LC | | In 20% increase |
| 15µg β-GC SOY + 15 µg β-LC | Normal | In 100% increase |
| TNBS with 15µg β-C | Normal | In 90% no change |
| | | In 10% increase |
| 15 µg β-LC | Normal | In 100% increase |

### Example 3

### Effect of mixtures of beta glycolipids on treatment of immune mediated colitis

To further investigate the possible use of beta glycolipids as a medication for colitis, the inventors next determined the effect of combinations of GC and LC on intra hepatic NKT regulatory lymphocytes and lymphocyte trapping.

Four groups of mice were studied. Immune mediated colitis was induced by intracolonic instillation of trinitrobenzenesulfonic-acid (TNBS) in all groups. Groups B-D were treated by daily administration of glucosylceramide (GC), lactosylceramide (LC), and a combination of both GC and LC (1:1 ratio), respectively. Mice in control group A received solvent alone. Mice were evaluated for macroscopic and microscopic colitis scores. The immune modulatory effect of beta-glycolipids was determined by FACS analysis of intrahepatic and intrasplenic lymphocytes for NKT, CD4 and CD8 markers, and by measurement of serum cytokine levels.

As shown by Table 4, administration of beta -glycolipids resulted in an increased intrahepatic/peripheral NKT ratio (p<0.05), and in a decreased peripheral/intrahepatic CD4+/CD8+ ratio. Beta-glycolipids led to a 43% increase in survival and significant alleviation of colitis with improvement in the macroscopic and microscopic scores (p<0.05), and to a decrease in serum IFNγ levels and IFNγ/IL-10 ratio in groups B-D compared with group A (p<0.01).

These results clearly indicate that combination of GC and LC lead to increased NKT regulatory lymphocyte redistribution, intrahepatic T lymphocyte trapping, and alleviated immune mediated colitis.

**Table 4**

| **Test group/ tested parameter** | **Control group A** | **GC group B** | **LC group C** | **IGL group D** |
|---|---|---|---|---|
| intrahepatic/peripheral NKT ratio | 1 | 7 | 15 | 4 |
| peripheral/intrahepatic CD4+/CD8+ ratio | 0.87 | 0.3 | 0.86 | 0.4 |
| % improvement in the macroscopic and microscopic scores compares to A | | 82 | 56 | 92 |
| IFNγ serum levels (pg/ml) | 437 | 83 | 58 | 184 |
| IFNγ/IL-10 ratio | 14 | 4 | 4 | 3 |

### Example 4

### Mixed Glycolipids in Colitis- determination of different mixture ratios

To test different combinations of the two most potent β-glycolipids:, β-glucocerebroside and β-lactosyl-ceramide, and to identify the most effective mixture combination, different quantitative ratio of both glycolipids were used (1:1, 1:10, and 1:100), using the murine model of colitis.

As summarized in Table 5, nine groups of C57BL mice were studied, each consisting of 10 mice. Colitis was induced by intracolonic installation of trinitrobenzenesulfonic acid (TNBS) on day 1 and 5 in group. Group A mice were fed regular chow diet. Groups B-F mice received oral (PO) daily of a mixture of β-GluC and β-LacC (GC and LC) in different ratio, as indicated by the table. Groups G-I received only β-LacC.

Mice were followed for macroscopic and microscopic colitis scores, as well as for different cell populations by FACS: CD4, CD8, NKT in spleen and liver (not pooled), and for serum cytokines IFNγ and IL4 by ELISA.

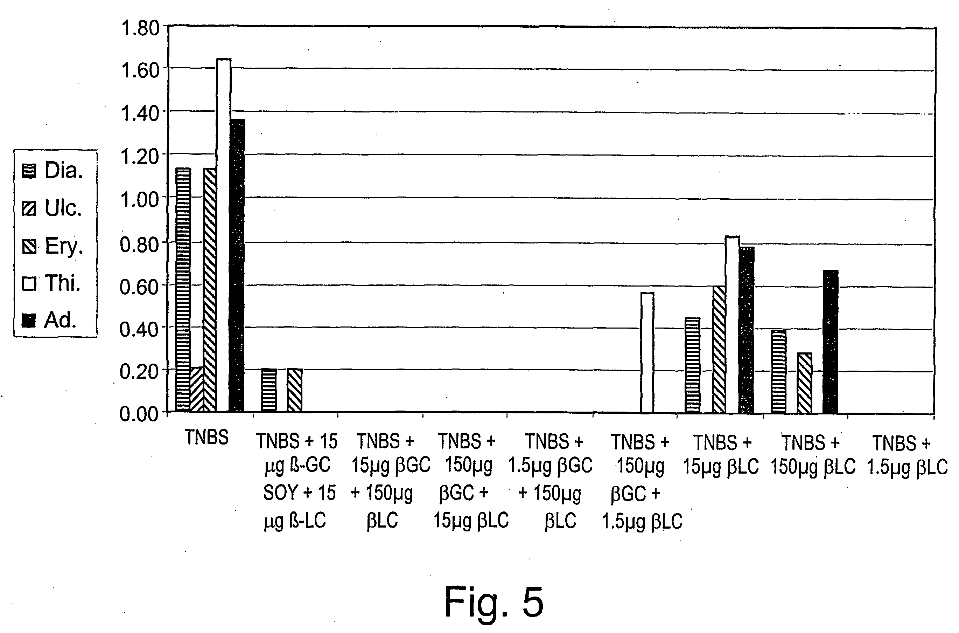

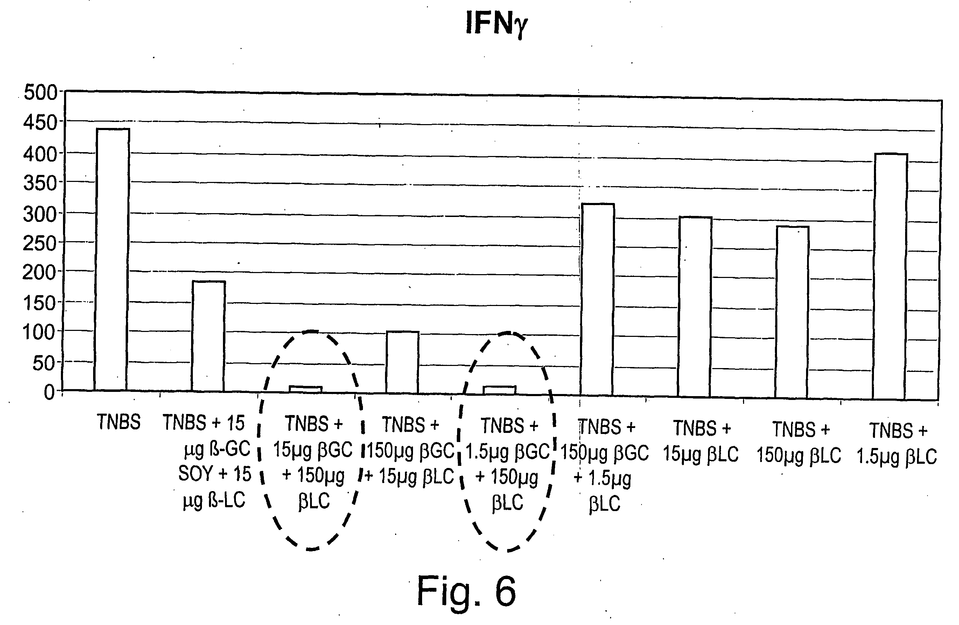

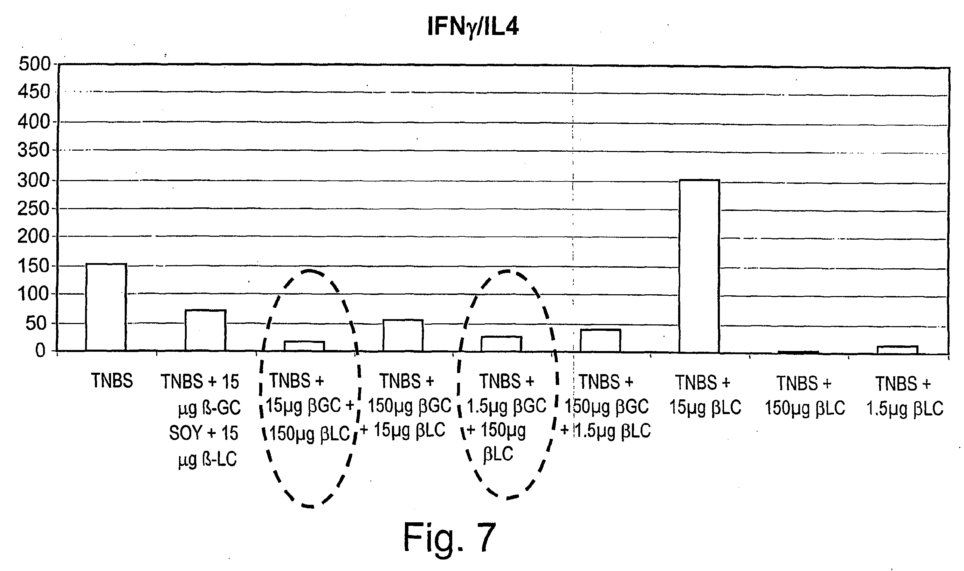

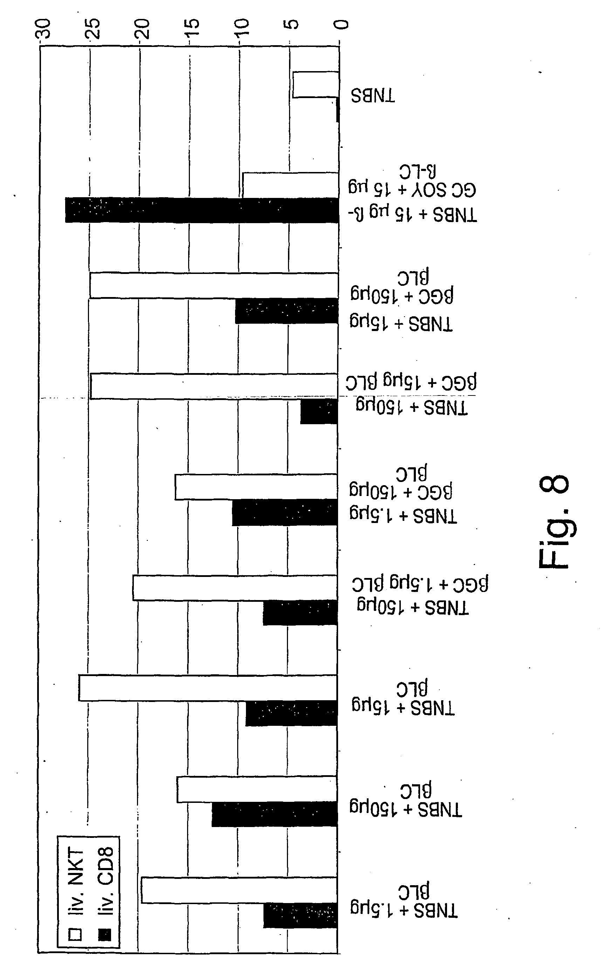

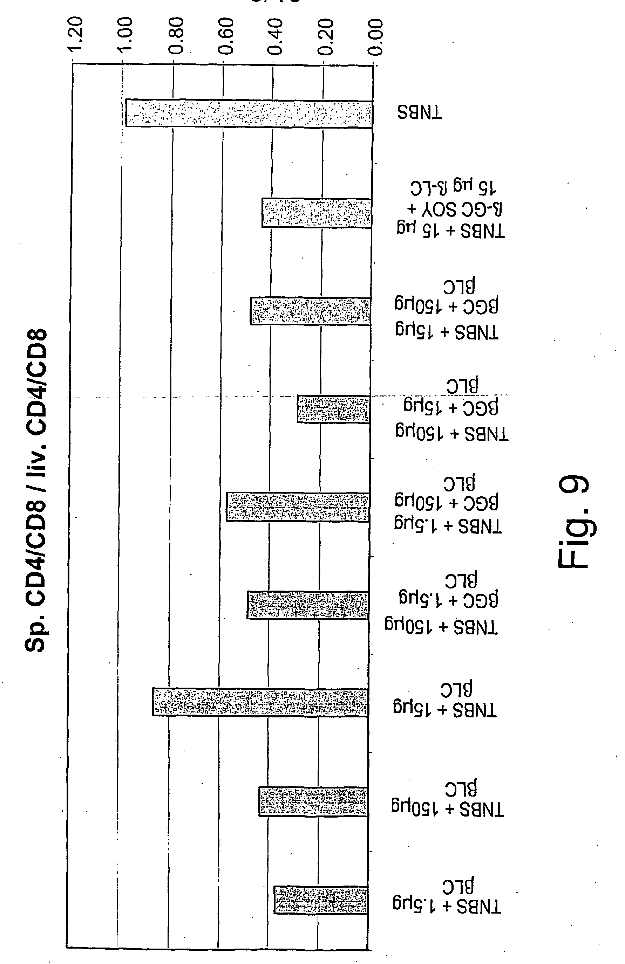

As shown by Figure 5, administration of different mixtures of β-GlucC and β-LacC in different ratios, led to significant amelioration of TNBS induced colitis, as indicated by the reduction in diarrhea, ulcers erythema, thickness and adhesions. This effect was especially significant in all three different mixture ratios, when compared to the effect of β-LacC alone. As shown by Figure 6, examination of serum IFNγ levels clearly indicated that both mixtures of 15µgGC+150µgLC and 1.5µgGC+150µgLC were the most effective in causing significant reduction of IFNγ levels. This reflects a beneficial shift toward a Th2 response. These two mixtures, and specifically the 15µgGC+150µgLC mixture, were most effective also in reducing the IFNγ/IL4 ratio, indicating the beneficial Th2 shift, as shown by Figure 7. The beneficial effect of these mixtures was associated also with increased intrahepatic NKT and CD8 lymphocytes as indicated by Figures 8 and 9. In summary, different combinations of GC and LC led to significant amelioration of TNBS induced colitis as also demonstrated by improved survival (40% increase), improved functional status and weight and clear shift to Th2 response. It should be indicated that most beneficial effects were caused by the 15µgGC+150µLC mixture.

**Table 5**

| **Group** | **Day 1 and 5** | **Day 1-9 feeding** |
|---|---|---|
| **A** | TNBS 0.5 mg X 2 | - |
| **B** | TNBS 0.5 mg X 2 | 15 µg β-GC with 15 µg β-LC |
| **C** | TNBS 0.5 mg X 2 | 15 µg β-GC with 150µg β-LC |
| **D** | TNBS 0.5 mg X 2 | 150 µg β-GC with 15 µg β-LC |
| **E** | TNBS 0.5 mg X 2 | 1.5 µg β-GC with 150 µg β-LC |
| **F** | TNBS 0.5 mg X 2 | 150 µg β-GC SOY with 1.5 µg β-LC |
| **G** | TNBS 0.5 mg X 2 | 15 µg β-LC |
| **H** | TNBS 0.5 mg X 2 | 150 µg β-LC |
| **I** | TNBS 0.5 mg X 2 | 1.5 µg β-LC |

### Example 5

### Use of β-glycolipids for the treatment of immune hepatocellular carcinoma

The immunomodulatory effect of different β-glycolipids and particularly of mixtures of GC+LC, demonstrated by the colitis model, encouraged the inventors to further investigate other immune-related disorders, such as hepatocellular carcinoma (HCC). Therefore, the clinical and immunological consequences of administration of β-glucosylceramide (GluC), β-lactosyl-ceramide (LacC), and β-galactosyl-ceramide (GalC) and of ceramide on hepatocellular carcinoma (HCC) were next examined, using mice transplanted with human Hep3B HCC. Five groups of athymic Balb/*c* mice, consisting of 8 mice each, were sublethally irradiated and transplanted with human Hep3B HCC, followed by daily intraperitoneal injections of GluC, LacC, GalC, ceramide (1.5 µg in 100µl PBS) or PBS (100µ) for 25 days. Animals were followed for tumor size and weight and for intrahepatic and intrasplenic lymphocyte subpopulations, serum cytokine levels and expression of STAT1, STAT4 and STAT6 in splenocytes. The different test groups are summarized in Table 6.

**Table 6**

| Group: | Treatment: |
|---|---|
| A | Hep3B HCC, 1.5 µg β-GluC |
| B | Hep3B HCC, 1.5 µg β-LacC |
| C | Hep3B HCC, 1.5 µg β-GalC |
| D | Hep3B HCC, 1.5 µg ceramide |
| E | Hep3B HCC, PBS control |

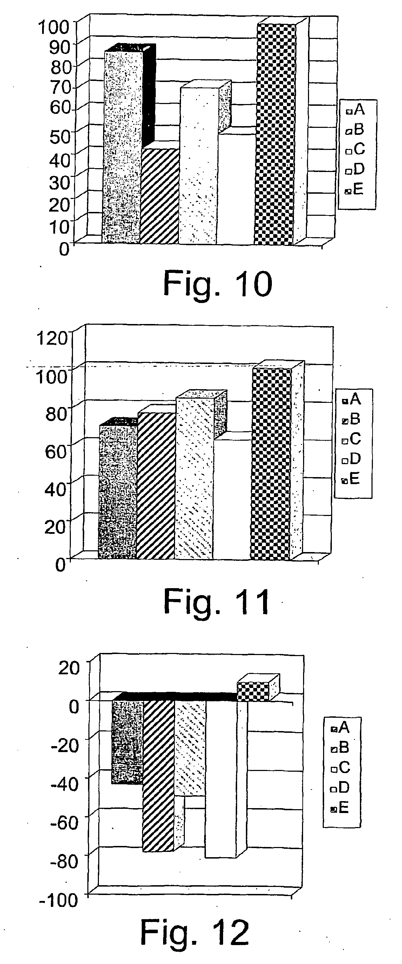

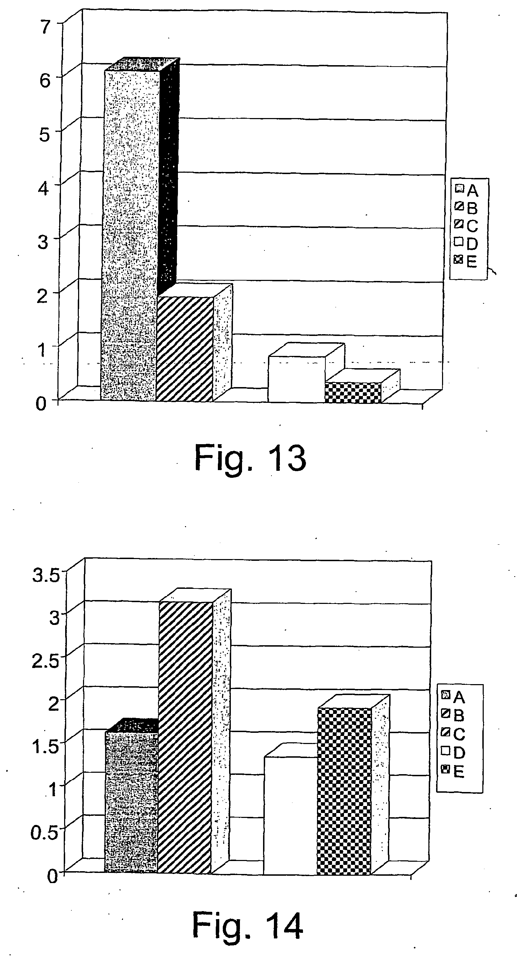

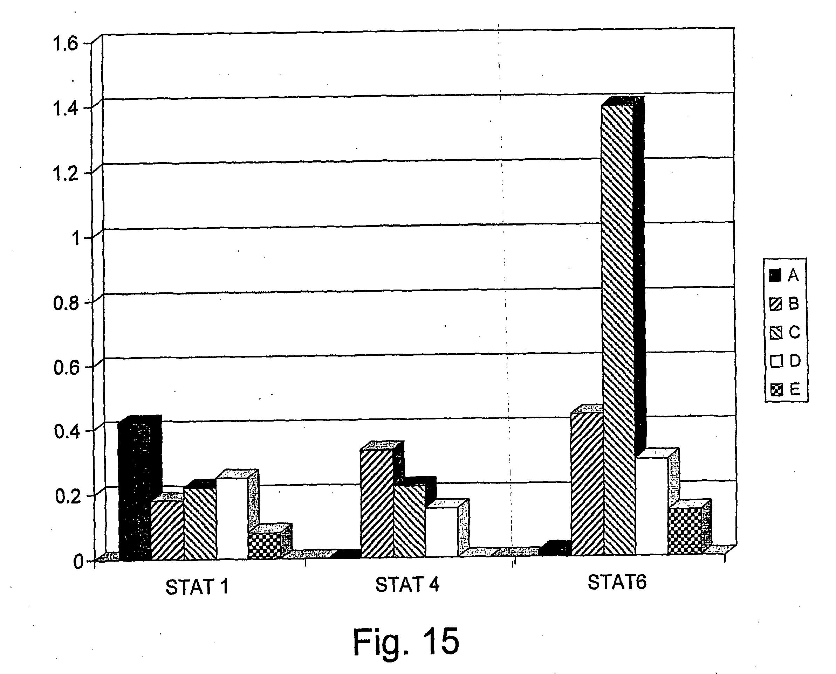

As shown in Figure 10, administration of β-glycolipids and ceramide resulted in marked suppression of HCC. Tumors developed in 87%, 43%, 71% and 50% of GluC, LacC, GalC and ceramide-treated animals, respectively, compared to 100% of controls. Figure 11 further shows that maximal tumor volume was 71.4, 77.9, 86.1 and 64.4 mm³ in GluC, LacC, GalC and ceramide-treated animals, respectively, compared to 101.14 mm³ in controls (p<0.05). Moreover, Figure 12 indicates a 43%, 78%, 49% and 81% reduction of tumor volume, in GluC, LacC, GalC and ceramide treated mice, respectively, in contrast to a 10% increase in tumor volume in controls (p<0.05). Body weight did not differ significantly among the groups. As shown in Figure 13, the beneficial effect of β-glycolipids was associated with increased intrahepatic NKT lymphocytes (hepatic/splenic NKT lymphocyte ratio 6.13, 1.94, 0.85 and 0.38 in groups A, B, D and E, respectively, p<0.05). Figure 14 shows that the effect of β-glycolipids was further associated with increased intrahepatic CD8 T lymphocyte trapping. As shown in Figure 15, STAT1 expression in splenocytes was increased in glycolipid-treated mice vs. controls, and STAT4 and STAT6 expression was increased in LacC-, GalC- and ceramide-treated animals. It should be noted that serum cytokine levels did not differ significantly between the groups (data not shown).

These results clearly demonstrate the feasibility of using β-glycolipids, for the treatment of other immune-related disorders such as HCC. Administration of β-glycolipids, resulted in suppression of HCC, accompanied by increased intrahepatic NKT lymphocytes, increased intrahepatic CD8 T lymphocyte trapping and increased expression of STAT1, STAT4 and STAT6 in splenocytes. These results suggest that α configuration of glycolipid sugars may not be essential for NKT cell-related anti tumor effects.

### Example 6

### Effect of mixtures of β-glycolipids on the treatment of immune hepatocellular carcinoma

The inventors further analyzed the effect of different combinations of β-glycolipids, and particularly of mixtures of GC and LC (IGL), which were shown effective in the colitis model, by using the murine HCC model. Athymic Balb/c mice (n=8/group) were sublethally irradiated and transplanted with human Hep3B HCC, followed by daily intraperitoneal injections of PBS, GC, LC or IGL (1.5µg in 100µl PBS, groups A, B, C and D, respectively) for 25 days. Animals were followed for serum α-fetoprotein (AFP) and for intrahepatic and intrasplenic lymphocyte subpopulations.

Administration of GC, LC and IGL resulted in reduced serum AFP, reflecting suppression of HCC, that was most prominent in GC-treated animals (34560 vs. 169600 ng/ml in groups B and A, respectively). The beneficial effect of β-glycolipids was associated with increased intrahepatic NKT lymphocytes (hepatic/splenic NKT lymphocyte ratio 0.38, 6.13, 1.94, and 3.41 in groups A, B, C and D, respectively, p<0.05).

Therefore, alteration of lipid rafts in splenocytes by β-glycolipids, may mediate the immune-modulatory anti tumor effect associated with suppression of HCC by these compounds.

### Example 7

### Effect of β-glycolipids and mixtures thereof on ConA induced hepatitis

The inventors next analyzed the effect of different β-glycolipids and mixtures thereof on ConA induced hepatitis, using the ConA injected C57/bl mice model.

Fourteen experimental and control groups, 12 mice per group, were studied (Table 7). Mice in experimental groups A-G were injected with ConA. Group A mice were administered a single intraperitoneal injection of 100 µl PBS two hours prior to IV administration of ConA. Mice in groups B, C, D, E and F, were administered a single intraperitoneal injection of β-glucosylceramide, β-galactosyloceramide, β-lactosylceramide, iGb3, Ceramide, and IGL (a 1:1 ratio of β-glucosylceramide and β-lactosylceramide), respectively (1 µg in 100 µl PBS) 2 hours prior to IV administration of ConA. Mice in groups H-N were similarly injected with the different glycolipids without ConA administration. Animals were sacrificed ten hours following glycolipid injections, 8 hours after injection of ConA.

The effect of treatment with different β-glycolipids on liver damage in all tested groups, was assessed by determination of serum aspartate aminotransferase (AST), alanine aminotransferase (ALT) levels and histological examination of liver specimens. Assessment of the effect of β-glycolipids on the immune response, was determined by FACS analysis of intrahepatic and intrasplenic lymphocytes for NKT markers and measurement of serum cytokine levels.

**Table 7**

| Group: | ConA | Treatment |
|---|---|---|
| A | + | PBS |
| B | + | β-glucosylceramide |
| C | + | β-galactosylceramide |
| D | + | β-lactosylceramide |
| E | + | iGb3 |
| F | + | Ceramide |
| G | + | IGL |
| H | - | PBS |
| I | - | β-glucosylceramide |
| J | - | β-galactosylceramide |
| K | - | β-lactosylceramide |
| L | - | iGb3 |
| M | - | Ceramide |
| N | - | IGL |

Evaluation of the effect of β-glycolipids on the immune response is demonstrated by Figures 16-18. The inventors first examined the effect on splenic and intrahepatic NKT lymphocytes. As shown by Figure 16A, administration of ConA was associated with a significant decrease of NKT lymphocytes number (22.55 to 1.66, Groups H and A, respectively, p<0.005). Among ConA-treated groups, administration of β glycolipids led to an increase in the number of intrahepatic NKT cells (groups B to G compare with group A, p < 0.002). Although variability was noted between the effects of the different glycolipids, none of these differences was significant.

Administration of glycolipids to naive mice (groups I to N) led to reduction of intrahepatic NKT cell number (22.5 up to 1.3; p<0.005, for group I as compared with I to N). No significant differences were noted between the different glycolipid. In the spleen, a statistically insignificant increase in the NKT lymphocyte number was observed in all β-glycolipid treated groups with and without the administration of ConA (Figure 16B).

The liver/spleen NKT ratio was increased in all groups treated with β3-glycolipids with the administration of ConA. β-glucosylceramide, β-lactosylceramide, and IGL had a different effect when compared to all other tested glycolipids, as demonstrated by Figure 16C. For all three substances, a significantly higher ratio was noted in naive animals 2.03, 2.44 and 2.16 for groups I, K and N, respectively, p<0.005, in comparison with groups J, L, and M). Similarly, β-glucosylceramide, β-lactosylceramide, and IGL exerted a different effect on the live/spleen NKT lymphoycte ratio in ConA treated groups. Mice in groups B, D, and G, had a relatively lower ratio compared to groups C, E and F, (1.16, 1.28, 1.46, compared with 1.79, 2.61, 2.91, respectively, p<0.005, for B, D, and G, compared with each of C, E and F).

The Effect of β-glycolipids on splenic and intrahepatic CD4 and CD8 lymphocytes was next examined, as demonstrated by Figure 17. β-glucosylceramide, β-lactosylceramide, and IGL significantly decreased the intrahepatic CD8 trapping in the liver as manifested by a significant decrease of the spleen to liver CD4/CD8 lymphocyte ratio (1.23, 1.38, and 1.24, for mice in groups B, D, and G, respectively, as compared with 1.53, 1.61, and 1.53 for groups C, E and F, respectively, p<0.005, for B, D, and G, compared with each of C, E and F).

A similar effect of decreased intrahepatic CD8 trapping was noted in naive animals administered with β-glucosylceramide, β-lactosylceramide, and IGL (Figure 17).

The effect of β-glycolipids on serum cytokine levels in conA induced hepatitis model is demonstrated by Figure 18. As shown by the Figure, serum IFNγ levels were significantly reduced in β-glucosylceramide, β lactosylceramide, and IGL treated animals (86.1, 126.8, and 188.8 pg/ml, for mice in groups B, D, and G, respectively, as compared with 1264, 1343 and 1051 for groups C, E and F, respectively, p<0.005, for B, D, and G, compared with each of C, E and F).

Serum IL-10 levels decreased in animals treated with β-glycolipids compared with those treated with ConA alone (p NS). No significant change was noted in serum IL-12 levels between treated and untreated groups.

The beneficial effect of β-glucosylceramide, β lactosylceramide, and IGL clearly demonstrated by these results, was associated with a decrease in the peripheral to intra hepatic CD4/CD8 lymphocyte ratio, suggesting a decrease in the relative intrahepatic CD8 lymphocytes. This effect was also associated with alteration of the Th1/Th2 cytokine paradigm, as noted by a decreased in serum IFNγ levels, which is considered to have a key role in the pathogenesis of ConA-induced hepatitis.

These results encouraged the inventors to further investigate the effect of different β-glycolipids on liver dmage using the ConA hepatitis model. Therefore, serum AST and ALT levels were determined for all tested groups as demonstrated by Figure 19A and B, respectively.

As shown by the Figure, administration of β-glucosylceramide (GC), β-lactosylceramide (LC), and the IGL mixture, significantly alleviated ConA induced hepatitis as manifested by a decrease of serum AST and ALT levels (182, 244, and 175 IU, for mice in groups B, D, and G, respectively, as compared with 1881 IU for non treated animals in groups A, p<0.005). Other beta glycolipids failed to exert a beneficial effect (1478, 1331 and 823 IU, for groups C, E and F, respectively, p<0.005, in comparison with groups B, D, and G).

Interestingly, some of the glycolipids that did not exert a beneficial effect in ConA treated animals, induced mild hepatitis in naive mice. Similar effects were noted on the AST serum levels.

As shown by Figure 20, histological liver damage was markedly attenuated in β-glucosylceramide, β-lactosylceramide, and IGL treated groups. Total liver score was decreased to 1.5, 1.75, and 1.16 for mice in groups B, D, and G, respectively, when compared with 6.0, in non-treated controls in group A, and 2.66, 3.0, and 2.33, in mice in groups C, E and F, respectively (p<0.005 for B, D, and G, compared with each of C, E and F).

These results clearly demonstrate the feasibility of using glycolipids and particularly, LC and mixture thereof, in the treatment of immune-related disorders such as the ConA induced hepatitis.

### Example 8

### β-Glycolipids for the treatment of non alcoholic steactohepatitis (NASH)

### Effect of β-glycolipids and mixtures thereof on diabetes

To evaluate the effect of different β-glycolipids and mixtures thereof on diabetes, 12 groups of C57bl mice, consisting of 12 mice each are studied. Groups A-F mice are ob/ob mice, whereas Groups G-K are C57bl mice. Groups A-E and Groups G-K mice are injected intraperitoneally with 1.5 µg in 100 µl PBS every other day for 14 days with the following β-glycolipids: GluC (groups A,G), LacC (groups B, H), GalC (groups C, I), ceramide (groups D, J) and a mixture of GluC and LacC (groups F, L). Group F and Group L naive ob/ob mice and naive C57bl mice, respectively, are left untreated and serve as controls.

On the 14^{th} day, glucose tolerance tests are performed on 6 mice from each group.

### Effect of orally administered β-glycolipids and mixtures thereof on NASH

To evaluate the effect of different β-glycolipids and mixtures thereof on the various metabolic and immunologic components of the NASH model, 12 groups of C57bl mice, consisting of 12 mice each are studied. Groups A-F mice are ob/ob mice, whereas Groups G-K are C57bl mice. Groups A-E and Groups G-K mice receive for 14 days oral daily amount of 15 µg of the following β-glycolipids: GluC (groups A,G), LacC (groups B, H), GalC (groups C, I); ceramide (groups D, J) and a mixture of GluC and LacC (groups F, L) . Group F and Group L naive ob/ob mice and naive C57bl mice, respectively, are left untreated and serve as controls.

On the 14^{th} day, glucose tolerance tests are performed on 6 mice from each group.

### The Effect of β-glycolipids on the hepatic fat content

To determine the effect of β-glycolipids and mixtures thereof on hepatic fat content, 12 groups of C57bl mice, consisting of 12 mice each are studied. Groups A-F mice are ob/ob mice, whereas Groups G-K are C57bl mice. Groups A-E and Groups G-K mice are injected intraperitoneally with 1.5 µg in 100 µl PBS every other day for 14 days with the following β-glycolipids: GluC (groups A,G), LacC (groups B, H), GalC (groups C, I), ceramide (groups D, J) and a mixture of GluC and LacC (groups F, L). Group F and Group L naive ob/ob mice and naive C57bl mice, respectively, are left untreated and serve as controls.

To determine hepatic fat content, mice of all test groups undergoing an abdominal MRI on day 14 of the experiment. Hepatic fat content is determined and described as the SI index IP-OP/IP. Liver size, in area, is also determined.

### Example 9

### Mixed β-glycolipids in diabetic pssammomys model

To determine the effect of different combinations of β-glycolipids, and particularly of a mixture of β-glucocerebroside and β-lactosyl-ceramide) on diabetes, metabolic syndrome and hepatic steatosis, the diabetic Passamon model was used. The sand rat *(Psammomys obesus),* a model of nutritionally-induced type II diabetes, develops significant hyperinsulinemia, hyperglycemia and hypertriglyceridemia on a high-energy diet. Three groups of five-month-old sand rats on high energy diets were studied (n=10 per group). Animals were treated by daily intraperitoneal injections of GC (group A), IGL (group B), or PBS (group C), for 25 days. On day 25, all psammomys are sacrificed and examined by the following analysis:

Determination of hepatic fat content and inflammation was performed by magnetic resonance imaging (MRI), examination of liver biopsies and measurement of serum Alanine aminotransferase (ALT) and Aspartate aminotransferase (AST) levels. Body weight and post prandial serum glucose, insulin, triglyceride and free fatty acid (FFA) levels were assessed. To determine the mechanism of the effect of IGL on the Th1/Th2 balance, expression of the transcription factors STAT1, STAT4, STAT5 and STAT6 was determined in splenocytes.

As shown by Table 8, combination of two β-glycolipids, GC and LC (IGL) significantly ameliorated the metabolic alterations in *P. obesus,* and showed better results than GC alone. Mean post prandial glucose levels decreased in treated animals (p<0.01, between groups B and C) along with a decrease in plasma insulin levels (p=0.017, between groups B and C), and plasma FFA (p<0.01). As clearly indicated by this table, amelioration of hepatic steatosis and steatohepatitis in liver biopsies, and normalization of serum ALT and AST levels were noted. Increased expression of STAT1 and 4 was observed in IGL-treated animals. However, no significant change was noted in body weight, liver weight, and plasma triglyceride levels.

These results clearly indicate that combination of two β-glycolipids significantly ameliorated the insulin resistance and the hepatic damage, along with a decrease in plasma FFA levels *in Psammoinys obesus.* Therefore, these naturally-occurring compounds are promising agents for treatment of both NAFLD and the metabolic syndrome.

**Table 8**

| **Test group/ Tested parameter** | **GC (group A)** | **IGL (group B)** | **PBS control (group C)** |
|---|---|---|---|
| **Mean post prandial glucose levels (mg%)** | 160 | 62 | 219 |
| **plasma insulin levels (mU/l)** | 66 | 36 | 111 |
| **plasma FFA (µmol/l)** | 368 | 172 | 665 |
| **hepatic fat (MRI SI index)** | 0.16 | 0.13 | 0.25 |
| **mean serum ALT (IU/L)** | 1283 | 150 | 954 |
| **mean serum AST (IU/L)** | 1137 | 177 | 1013 |

### Example 10

### Amelioration of non alcoholic fatty liver disease (NAFLD) and the metabolic syndrome in diabetic Cohen rats is associated with reduced pancreatic injury

To determine the effect of a mixture of β-glucocerebroside and β-lactosyl-ceramide, on diabetes, hepatic steatosis and metabolic syndrome, the Cohen rat model was used by the inventors. The Cohen rat is a lean, non-insulin resistant model of type 2 diabetes that features zone 1 and 2 mixed micro and macrovesicular steatosis and elevated serum transaminases.

Four groups of Cohen rats were studied (n=10/group). Animals were treated by daily intraperitoneal injections of GC (group A), LC (group B), IGL (group C), or PBS (group D) for 45 days. Assessment of NAFLD was performed by MR imaging, examination of liver biopsies and measurement of serum transaminases. Metabolic follow up parameters included body weight, oral glucose tolerance test (OGTT), serum lipids and pancreatic histology. Immune modulation was assessed by FACS analysis of intrahepatic and intrasplenic lymphocytes.

As summarized clearly by Table 9, administration of LC and IGL resulted in reduced hepatic fat content and serum transaminases (ALT and AST). In the OGTT, glycolipids significantly reduced the area under the glucose curve and increased the area under the insulin curve. Treatment with IGL resulted in significantly reduced serum triglycerides. In contrast to the marked fat infiltration, atrophy and fibrosis characteristic of control group pancreata (group D), pancreatic histology was markedly improved in all treated groups. β-glycolipid treatment did not affect body weight, liver histology or serum cholesterol. Increased intrahepatic CD8 T lymphocyte trapping in LC and IGL-treated animals and increased intrahepatic/splenic NKT cell ratio in GC-treated animals were observed.

These results show that combination of GC and LC had a therapeutic benefit exceeding that of either glycolipid alone, and was associated with marked amelioration of pancreatic injury and improved insulin secretion in treated animals. The alteration of lymphocyte subpopulations in treated groups may suggest an additional immune modulatory effect. Therefore, the use of this model also clearly indicates that administration of β glycolipid combinations holds promise as a therapeutic modality for NAFLD and the metabolic syndrome.

**Table 9**

| **Test group/ Tested parameter** | **GC (group A)** | **LC (group B)** | **IGL (group C)** | **PBS (group D)** | |
|---|---|---|---|---|---|
| **hepatic fat content (MRI SI index** | 0.23 | 0.15 | 0.14 | 0.19 | P< 0.05 |
| **mean serum ALT (IU/L)** | 268 | 251 | 174 | 301 | P< 0.05 |
| **mean serum AST (U/L)** | 75 | 59 | 45 | 83 | P< 0.05 |
| **OGTT (mg/dl/120')** | 25642 | 25413 | 23757 | 30333 | P< 0.45 |
| **Insulin (µg/l/120')** | 100 | 79 | 131 | 74 | P< 0.05 |

## Claims

1. A composition comprising a mixture of a naturally occuring β-glucosyl ceramide and a naturally occuring β-lactosyl ceramide for the treatment of an immune related disorder.

2. A composition according to claim 1 for use in the treatment of an immune-related disorder, wherein said treatment comprises modulation of the Th1/Th2 cell balance toward anti-inflammatory cytokine producing cells by increasing the intracellular extracellular or serum levels of a naturally occurring β-glycolipid in a subject suffering from said immune-related disorder, said modulation being mediated by at least one component of said subject's immune system.

3. The composition according to claim 1 or 2, wherein said mixture comprises β-glucosyl-ceramide and β-lactosyl-ceramide at a quantitative ratio between 1:1 to 1:1000.

4. The composition according to claim 3, wherein said mixture comprises β-glucosyl-ceramide and β-lactosyl-ceramide at a quantitative ratio of 1:10.

5. The composition according to claim 4, wherein said mixture comprises between about 0.5 to 5 mg per kg of body weight of β-glucosyl-ceramide and between 5 to 50 mg per kg of body weight of β-lactosyl-ceramide at a quantitative ratio of 1:10.

6. The composition according to claim 5, wherein said mixture comprises 0.75 mg per kg of body weight of β-glucosyl-ceramide and 7.5 mg per kg of body weight of β-lactosyl-ceramide.

7. A therapeutic composition for the treatment of an immune-related disorder in a mammalian subject comprising as an active ingredient a mixture of a naturally occuring β-glucosyl ceramide and a naturally occuring β-lactosyl ceramide.

8. A composition according to claim 7 for the modulation of the Th1/Th2 cell balance toward the Th2 anti-inflammatory cytokine producing cells.

9. The composition according to claims 7 or 8, optionally further comprising any one of:
a. antigens associated with said immune-related disorder;
b. at least one of liver-associated cells of tolerized or non-tolerized subjects suffering from said immune-related disorder or of said subject;
c. at least one of cytokines, adhesion molecules or any combination thereof;
d. antigen presenting cells; or
e. a combination of any (a), (b), (c) or (d).

10. The composition according to claim 9, wherein said mixture comprises β-glucosyl-ceramide and β-lactosyl-ceramide at a quantitative ratio between 1:1 to 1:1000.

11. The composition according to claim 10, wherein said mixture comprises β-glucosyl-ceramide and β-lactosyl-ceramide at a quantitative ratio of 1:10.

12. The composition according to claim 11, wherein said mixture comprises between about 0.5 to 5 mg per kg of body weight of β-glucosyl-ceramide and between 5 to 50 mg per kg of body weight of β-lactosyl-ceramide at a quantitative ratio of 1:10.

13. The composition according to claim 12, wherein said mixture comprises 0.75 mg per kg of body weight of β-glucosyl-ceramide and 7.5 mg per kg of body weight of β-lactosyl-ceramide.

## Patentansprüche

1. Zusammensetzung, umfassend ein Gemisch aus einem natürlich vorkommenden β-Glucosylceramid und einem natürlich vorkommenden β-Lactosylceramid zur Behandlung einer mit dem Immunsystem assoziierten Störung.

2. Zusammensetzung nach Anspruch 1 zur Verwendung bei der Behandlung einer mit dem Immunsystem assoziierten Störung, wobei die Behandlung eine Modulation des Th1/Th2-Zellgleichgewichts hin zu entzündungshemmendes Cytokin produzierenden Zellen durch Erhöhung der intrazellulären, extrazellulären oder Serumspiegel eines natürlich vorkommenden β-Glycolipids bei einem Individuum umfasst, das an der mit dem Immunsystem assoziierten Störung leidet, wobei die Modulation durch mindestens eine Komponente des Immunsystems des Individuums vermittelt wird.

3. Zusammensetzung nach Anspruch 1 oder 2, wobei das Gemisch β-Glucosylceramid und β-Lactosylceramid in einem Mengenverhältnis zwischen 1:1 bis 1:1000 umfasst.

4. Zusammensetzung nach Anspruch 3, wobei das Gemisch β-Glucosylceramid und β-Lactosylceramid in einem Mengenverhältnis von 1:10 umfasst.

5. Zusammensetzung nach Anspruch 4, wobei das Gemisch zwischen etwa 0,5 bis 5 mg pro kg Körpergewicht an β-Glucosylceramid und zwischen 5 bis 50 mg pro kg Körpergewicht an β-Lactosylceramid in einem Mengenverhältnis von 1:10 umfasst.

6. Zusammensetzung nach Anspruch 5, wobei das Gemisch 0,75 mg pro kg Körpergewicht an β-Glucosylceramid und 7,5 mg pro kg Körpergewicht an β-Lactosylceramid umfasst.

7. Therapeutische Zusammensetzung zur Behandlung einer mit dem Immunsystem assoziierten Störung in einem Säuger-Individuum, die als Wirkstoff ein Gemisch aus einem natürlich vorkommenden β-Glucosylceramid und einem natürlich vorkommenden β-Lactosylceramid umfasst.

8. Zusammensetzung nach Anspruch 7 zur Modulation des Th1/Th2-Zellgleichgewichts hin zu den entzündungshemmenden Cytokin produzierenden Th2-Zellen.

9. Zusammensetzung nach den Ansprüchen 7 oder 8, die gegebenenfalls des Weiteren ein Beliebiges aus Folgenden umfasst:
a. Antigene, die mit der mit dem Immunsystem assoziierten Störung in Zusammenhang stehen;
b. mindestens eine aus Leber-assoziierten Zellen von Individuen mit entwickelter Immuntoleranz oder nicht entwickelter Immuntoleranz, die an der mit dem Immunsystem assoziierten Störung leiden, oder von dem Individuum;
c. mindestens eines aus Cytokinen, Adhäsionsmolekülen oder einer beliebigen Kombination daraus;
d. Antigen-präsentierende Zellen; oder
e. eine beliebige Kombination aus (a), (b), (c) oder (d).

10. Zusammensetzung nach Anspruch 9, wobei das Gemisch β-Glucosylceramid und β-Lactosylceramid in einem Mengenverhältnis zwischen 1:1 bis 1:1000 umfasst.

11. Zusammensetzung nach Anspruch 10, wobei das Gemisch β-Glucosylceramid und β-Lactosylceramid in einem Mengenverhältnis von 1:10 umfasst.

12. Zusammensetzung nach Anspruch 11, wobei das Gemisch zwischen etwa 0,5 bis 5 mg pro kg Körpergewicht an β-Glucosylceramid und zwischen 5 bis 50 mg pro kg Körpergewicht an β-Lactosylceramid in einem Mengenverhältnis von 1:10 umfasst.

13. Zusammensetzung nach Anspruch 12, wobei das Gemisch 0,75 mg pro kg Körpergewicht an β-Glucosylceramid und 7,5 mg pro kg Körpergewicht an β-Lactosylceramid umfasst.

## Revendications

1. Composition comprenant un mélange d'un β-glucosyl céramide naturel et d'un β-lactosyl céramide naturel pour le traitement d'un trouble immunitaire.

2. Composition selon la revendication 1 pour une utilisation dans le traitement d'un trouble immunitaire, où ledit traitement comprend une modulation de la balance de cellules Th1/Th2 vers des cellules productrices de cytokine anti-inflammatoire en augmentant les taux intracellulaire extracellulaire ou sérique d'un β-glycolipide naturel chez un sujet souffrant dudit trouble immunitaire, ladite modulation étant médiée par au moins un composant dudit système immunitaire du sujet.

3. Composition selon la revendication 1 ou 2, dans laquelle ledit mélange comprend un β-glucosyl-céramide et un β-lactosyl-céramide dans un rapport quantitatif entre 1/1 et 1/1000.

4. Composition selon la revendication 3, dans laquelle ledit mélange comprend un β-glucosyl-céramide et un β-lactosyl-céramide dans un rapport quantitatif de 1/10.

5. Composition selon la revendication 4, dans laquelle ledit mélange comprend entre environ 0,5 à 5 mg par kilo en poids corporel de β-glucosyl-céramide et entre 5 et 50 mg par kilo en poids corporel de β-lactosyl-céramide dans un rapport quantitatif de 1/10.

6. Composition selon la revendication 5, dans laquelle ledit mélange comprend 0,75 mg par kilo en poids corporel de β-glucosyl-céramide et 7,5 mg par kilo en poids corporel de β-lactosyl-céramide.

7. Composition thérapeutique pour le traitement d'un trouble immunitaire chez un sujet mammifère comprenant en tant que principe actif un mélange d'un β-glucosyl-céramide naturel et d'un β-lactosyl-céramide naturel.

8. Composition selon la revendication 7, pour la modulation de la balance de cellules Th1/Th2 vers des cellules productrices de cytokine anti-inflammatoire Th2.

9. Composition selon les revendications 7 ou 8, comprenant facultativement en outre l'un quelconque parmi :
a. des antigènes associés audit trouble immunitaire ;
b. au moins une cellule associée au foie des sujets tolérants ou non-tolérants souffrant dudit trouble immunitaire ou dudit sujet ;
c. au moins une des cytokines, molécules d'adhésion, ou toute combinaison de celles-ci ;
d. cellules présentatrices d' antigène ; ou
e. une combinaison de (a), (b), (c), ou (d).

10. Composition selon la revendication 9, dans laquelle ledit mélange comprend un β-glucosyl-céramide et un β-lactosyl-céramide dans un rapport quantitatif entre 1/1 et 1/1000.

11. Composition selon la revendication 10, dans laquelle ledit mélange comprend un β-glucosyl-céramide et un β-lactosyl-céramide dans un rapport quantitatif de 1/10.

12. Composition selon la revendication 11, dans laquelle ledit mélange comprend entre environ 0,5 à 5 mg par kilo en poids corporel de β-glucosyl-céramide et entre 5 et 50 mg par kilo en poids corporel de β-lactosyl-céramide dans un rapport quantitatif de 1/10.

13. Composition selon la revendication 12, dans laquelle ledit mélange comprend 0,75 mg par kilo en poids corporel de β-glucosyl-céramide et 7,5 mg par kilo en poids corporel de β-lactosyl-céramide.
